(19) Europäisches Patentamt
European Patent Office
Office européen des brevets

(11) Publication number: 0 349 063 B1

# (12) EUROPEAN PATENT SPECIFICATION

(45) Date of publication of patent specification: 13.09.95

(51) Int. Cl.[6]: C07D 309/30, C07C 69/757, A61K 31/365, A61K 31/22

(21) Application number: 89201642.9

(22) Date of filing: 22.06.89

## (54) 5-oxygenated HMG-COA reductase inhibitors.

(30) Priority: 29.06.88 US 213010
13.03.89 US 322398
29.09.88 US 250646

(43) Date of publication of application:
03.01.90 Bulletin 90/01

(45) Publication of the grant of the patent:
13.09.95 Bulletin 95/37

(84) Designated Contracting States:
AT BE CH DE FR GB IT LI LU NL SE

(56) References cited:
JP-A-59 186 972

DERWENT JAPANESE PATENT REPORT, vol. 9, no. 45 (C-268)[1768], 26th February1985; & JP-A-59 186 972 (SUNTORY K.K.) 23-10-1984. (See also english translation)

Organic Chemistry, Allinger, pages 474-475

(73) Proprietor: MERCK & CO. INC.
126, East Lincoln Avenue
P.O. Box 2000
Rahway
New Jersey 07065-0900 (US)

(72) Inventor: Duggan, Mark E.
625 E. Lancaster Avenue
C101, Wynnewood, PA 19096 (US)
Inventor: Hartman, George D.
1529 Tennis Circle
Lansdale, PA 19446 (US)

(74) Representative: Cole, William Gwyn et al
European Patent Department
Merck & Co., Inc.
Terlings Park
Eastwick Road
Harlow
Essex CM20 2OR (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

## Description

BACKGROUND OF THE INVENTION

Hypercholesterolemia is known to be one of the prime risk factors for ischemic cardiovascular disease, such as arteriosclerosis. Bile acid sequestrants have been used to treat this condition; they seem to be moderately effective but they must be consumed in large quantities , i.e. several grams at a time and they are not very palatable.

MEVACOR® (lovastatin), now commercially available, is one of a group of very active antihypercholesterolemic agents that function by limiting cholesterol biosynthesis by inhibiting the enzyme, HMG-CoA reductase. In addition to the natural fermentation products, mevastatin and lovastatin, there are a variety of semi-synthetic and totally synthetic analogs thereof.

The naturally occurring compounds and their semi-synthetic analogs have the following general structural formulae:

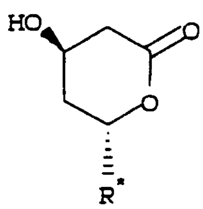

OR

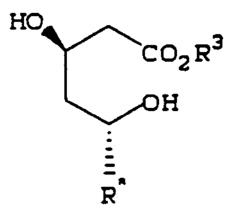

wherein:

$R^3$ is hydrogen, $C_{1-5}$ alkyl or $C_{1-5}$ alkyl substituted with a member of the group consisting of phenyl, dimethylamino, or acetylamino; and

$R^*$ is

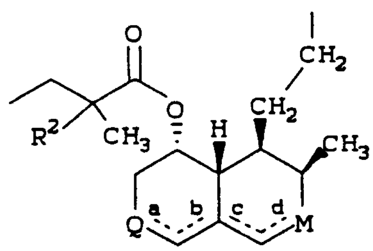

wherein

Q is

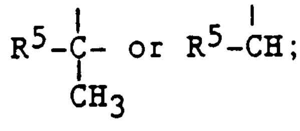

$R^5$ is H or OH;

M is

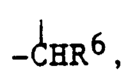

$R^6$ is hydrogen or hydroxy;

$R^2$ is hydrogen or methyl; and a, b, c, and d represent single bonds, one of a, b, c or d represents a double bond, or both a and c or both b and d represent double bonds provided that when a is a double bond, Q is

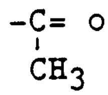
or
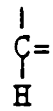

and when d is a double bond, M is

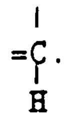

U.S. Patent 4,517,373 discloses semi-synthetic hydroxy containing compounds represented by the above general formula wherein R* is

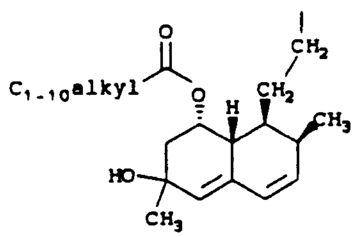
AND
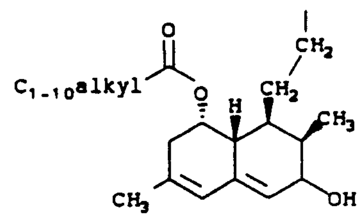

U.S. Patent 4,537,859 and U.S. Patent 4,448,979 also disclose semi-synthetic hydroxy-containing compounds represented by the above general formula wherein R* is

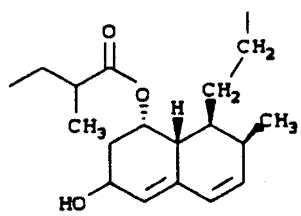
AND
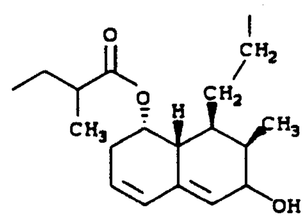

These compounds are prepared by the action of certain microorganisms on the corresponding non-hydroxylated substrates. One such organism described in U.S. 4,537,859 is of the genus Nocardia.

U.K. Patent 2,075,013 discloses semi-synthetic hydroxy containing compounds represented by the above general formula wherein R* is:

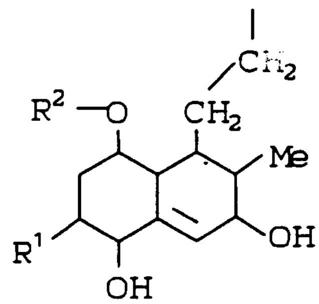

wherein $R^1$ is H or Me, and $R^2$ is H or acyl.

U.S. Patent Application Serial No. 048,136 filed May 15, 1987 discloses 6-substituted compounds of the above general formula wherein R* is:

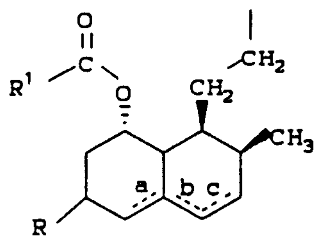

wherein R is $CH_2OH$,

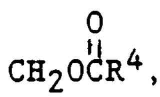

$CO_2R^7$ or

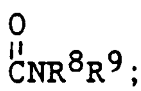

and $R^1$, $R^4$, $R^7$, $R^8$ and $R^9$ are broadly defined organic moieties.

U.S. Patents 4,604,472 and 4,733,003 disclose compounds of the above formula wherein R* is:

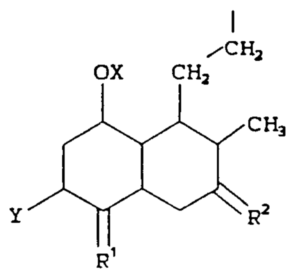

wherein X represents a hydrogen atom or a 2-methylbutyryl group, Y represents a hydrogen atom or a methyl group and $R^1$ and $R^2$ are the same or different and each represents an oxygen atom or a group of formula $=N\text{-}OR^3$ where $R^3$ is a hydrogen or alkyl moiety.

Japanese patent specification no. 59-186972 discloses compounds of the formula

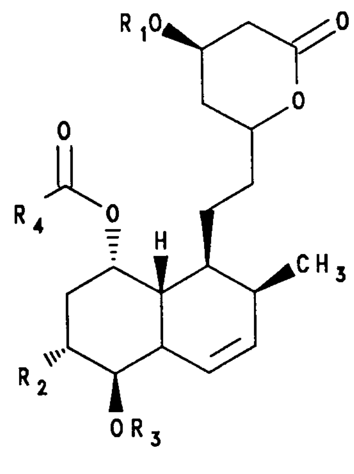

wherein $R_1$ is H; $R_2$ is lower alkyl; $R_3$ is H, tert-butyldimethylsilyl, triethylsilyl or benzyl; and $R_4$ is lower alkyl. These compounds are described as being useful intermediates for the preparation of mevinolin and mevinolinic acid.

DETAILED DESCRIPTION OF THE INVENTION

This invention relates to HMG-CoA reductase inhibitors of formulae (I) and (II):

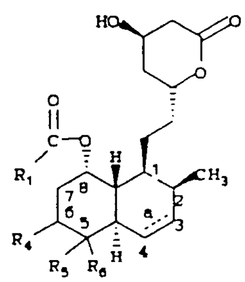

(I)

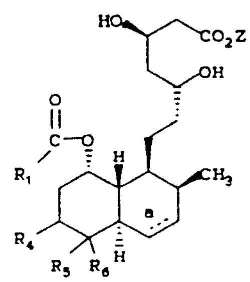

(II)

(Numbering relates to positions in tetrahydronaphthyl ring).

wherein:

$R_1$ is selected from:

(1) $C_{1-10}$ alkyl;

(2) substituted $C_{1-10}$ alkyl in which one or more substituent(s) is selected from

(a) halogen,

(b) hydroxy,

(c) $C_{1-10}$ alkoxy,

(d) $C_{1-5}$ alkoxycarbonyl,

(e) $C_{1-5}$ acyloxy,

(f) $C_{3-8}$ cycloalkyl,

(g) phenyl,

(h) substituted phenyl in which the substituents are X and Y,

(i) $C_{1-10}$ alkylS(0)$_n$ in which n is 0 to 2,

(j) $C_{3-8}$ cycloalkylS(0)$_n$,

(k) phenylS(0)$_n$,

(l) substituted phenylS(0)$_n$ in which the substituents are X and Y, and

(m) oxo;

(3) $C_{1-10}$ alkoxy;
(4) $C_{2-10}$ alkenyl;
(5) $C_{3-8}$ cycloalkyl;
(6) substituted $C_{3-8}$ cycloalkyl in which one substituent is selected from
(a) $C_{1-10}$ alkyl
(b) substituted $C_{1-10}$ alkyl in which the substituent is selected from
(i) halogen,
(ii) hydroxy,
(iii) $C_{1-10}$ alkoxy,
(iv) $C_{1-5}$ alkoxycarbonyl,
(v) $C_{1-5}$ acyloxy,
(vi) phenyl,
(vii) substituted phenyl in which the substituents are X and Y
(viii) $C_{1-10}$ alkylS(O)$_n$,
(ix) $C_{3-8}$ cycloalkylS(O)$_n$,
(x) phenylS(O)$_n$,
(xi) substituted phenylS(O)$_n$ in which the substituents are X and Y, and
(xii) oxo,
(c) $C_{1-10}$ alkylS(0)$_n$,
(d) $C_{3-8}$ cycloalkylS(0)$_n$,
(e) phenylS(0)$_n$,
(f) substituted phenylS(0)$_n$ in which the substituents are X and Y,
(g) halogen,
(h) hydroxy,
(i) $C_{1-10}$ alkoxy,
(j) $C_{1-5}$ alkoxycarbonyl,
(k) $C_{1-5}$ acyloxy,
(l) phenyl, and
(m) substituted phenyl in which the substituents are X and Y;
(7) phenyl;
(8) substituted phenyl in which the substituents are X and Y;
(9) amino;
(10) $C_{1-5}$ alkylamino;
(11) di($C_{1-5}$ alkyl)amino;
(12) phenylamino;
(13) substituted phenylamino in which the substituents are X and Y;
(14) phenyl $C_{1-10}$ alkylamino;
(15) substituted phenyl $C_{1-10}$ alkylamino in which the substituents are X and Y;
(16) a member selected from
(a) piperidinyl,
(b) pyrrolidinyl,
(c) piperazinyl,
(d) morpholinyl, and
(e) thiomorpholinyl; and
(17) $R_{10}S$ in which $R_{10}$ is selected from
(a) $C_{1-10}$ alkyl,
(b) phenyl, and
(c) substituted phenyl in which the substituents are X and Y;

$R_4$ is;
(1) hydrogen;
(2) $C_{1-10}$ alkyl; and
(3) substituted $C_{1-10}$ alkyl in which one or more substituents is selected from
(a) halogen,
(b) hydroxy,
(c) $C_{1-10}$ alkoxy
(d) $C_{1-5}$ alkoxycarbonyl,
(e) $C_{1-5}$ alkylacyloxy,
(f) phenylacyloxy,

(g) phenoxycarbonyl,
(h) phenyl $C_{1-5}$ alkylacyloxy,
(i) phenyl $C_{1-5}$ alkoxy,
(j) amino,
(k) $C_{1-5}$ alkylamino,
(l) di($C_{1-5}$ alkyl)amino,
(m) phenylamino,
(n) substituted phenylamino in which the substituents are X and Y;
(o) phenyl $C_{1-5}$ alkylamino,
(p) substituted phenyl $C_{1-5}$ alkylamino in which the substituents are X and Y,
(q) $C_{3-8}$ cycloalkyl,
(r) phenyl,
(s) substituted phenyl in which the substituents are X and Y,
(t) phenylS$(O)_n$,
(u) substituted phenyl S$(O)_n$ in which the substituents are X and Y,
(v) phenyl $C_{1-5}$ alkyl S$(O)_n$,
(w) $C_{1-5}$ alkylS$(O)_n$;
(x) phenylaminoacyloxy,
(y) $C_{1-5}$alkylaminoacyloxy,
(z) $C_{1-5}$alkylacylamino,
(aa) di(phenyl$C_{1-5}$alkyl)phosphonyl
(bb) di($C_{1-5}$alkyl)phosphinyl

(4) $R_4$ together with the carbon atom to which it is attached represents a $C_{3-8}$ carbocyclic ring;

$R_5$ and $R_6$ independently are H, OH, $OR_7$ or $R_5$ and $R_6$ together with the carbon to which they are attached represent C=O or $R_5$ and $R_6$ together with the carbon to which they are attached represent a carbocyclic ring of 3 to 7 atoms; provided that when $R_5$ is H, $R_6$ is OH or $OR_7$, and when $R_5$ is OH, $R_6$ is H, and when $R_5$ is $OR_7$, $R_6$ is H;

$R_7$ is

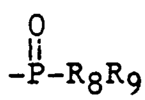, 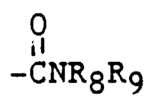, or 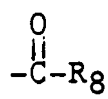, 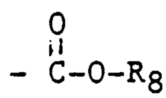,

phenyl$C_{1-3}$alkyl, $C_{1-5}$alkyl;

$R_8$ and $R_9$ independently are H, $C_{1-3}$alkyl, phenyl$C_{1-3}$alkyl or aryl wherein aryl is phenyl naphthyl, pyridyl, furanyl, thienyl or phenyl, naphthyl, pyridyl, furanyl or thienyl substituted with groups X and Y provided that when $R_7$ is

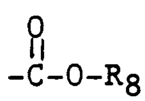,

$R_8$ is not H and when $R_7$ is

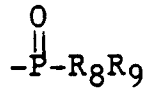

neither $R_8$ nor $R_9$ is H;

X and Y are independently selected from:

a) OH,
b) halogen,
c) trifluoromethyl,
d) $C_{1-3}$alkoxy,

e) $C_{1-3}$alkylcarbonyloxy,
f) phenylcarbonyloxy,
g) $C_{1-3}$alkoxycarbonyl,
h) phenyloxycarbonyl,
i) hydrogen;
j) $C_{1-5}$alkyl;

Z is selected from
(1) hydrogen;
(2) $C_{1-5}$alkyl;
(3) substituted $C_{1-5}$alkyl in which the substituent is selected from
(a) phenyl,
(b) dimethylamino, and
(c) acetylamino, and
(4) 2,3 hydroxypropyl;

halogen is Cl or F;

a is a single bond or a double bond; provided that when a is a double bond and $R_5$ or $R_6$ is OH, the configuration of the 5-position of the naphthyl ring is 5(R); or a pharmaceutically acceptable salt thereof.

Except where specifically defined to the contrary, the terms "alkyl", "alkenyl", "acyl" "aryloxy" and "alkoxy" include both the straight-chain and branched chain species of the term.

One embodiment of this invention is the class of compounds of formulae (I) and (II) wherein:

$R_1$ is selected from:
(1) $C_{1-10}$ alkyl;
(2) substituted $C_{1-10}$ alkyl in which one or more substituent(s) is selected from
(a) halogen,
(b) hydroxy,
(c) $C_{1-10}$ alkoxy,
(d) $C_{1-5}$ alkoxycarbonyl,
(e) $C_{1-5}$ acyloxy,
(f) $C_{3-8}$ cycloalkyl,
(g) phenyl,
(h) substituted phenyl in which the substituents are X and Y, and
(i) oxo;
(3) $C_{3-8}$ cycloalkyl;
(4) substituted $C_{3-8}$ cycloalkyl in which one substituent is selected from
(a) $C_{1-10}$ alkyl,
(b) substituted $C_{1-10}$ alkyl in which the substituent is selected from
(i) halogen,
(ii) hydroxy,
(iii) $C_{1-10}$ alkoxy
(iv) $C_{1-5}$ acyloxy,
(v) $C_{1-5}$ alkoxycarbonyl,
(vi) phenyl,
(vii) substituted phenyl in which the substituents are X and Y, and
(viii) oxo,
(c) halogen,
(d) hydroxy,
(e) $C_{1-10}$ alkoxy,
(f) $C_{1-5}$ alkoxycarbonyl,
(g) $C_{1-5}$ acyloxy,
(h) phenyl,
(i) substituted phenyl in which the substituents are X and Y;
(5) phenylamino;
(6) substituted phenylamino in which the substituents are X and Y;
(7) phenyl$C_{1-10}$alkylamino; and
(8) substituted phenyl $C_{1-10}$ alkylamino in which the substituents are X and Y;

$R_4$ is;

(1) hydrogen;
(2) $C_{1-10}$ alkyl;
(3) substituted $C_{1-10}$ alkyl in which one or more substituents is selected from:
(a) halogen,
(b) hydroxy,
(c) amino;
(4) $CH_2R_{12}$ in which $R_{12}$ is selected from:
(a) $C_{1-5}$ alkoxy,
(b) ($C_{1-5}$) alkoxy carbonyl,
(c) $C_{1-5}$ alkylacyloxy,
(d) phenylacyloxy,
(e) phenoxycarbonyl,
(f) phenyl$C_{1-5}$alkyl,
(g) phenyl$C_{1-5}$alkoxy
(h) $C_{1-5}$alkylamino,
(i) di($C_{1-5}$alkyl)amino,
(j) phenylamino,
(k) substituted phenylamino in which the substituents are X and Y,
(l) phenyl $C_{1-5}$alkylamino,
(m) substituted phenyl $C_{1-5}$ alkyl amino in which the substituents are X and Y,
(n) $C_{3-8}$ cycloalkyl,
(o) phenyl,
(p) substituted phenyl in which the substituents are X and Y,
(q) phenyl$S(O)_n$
(r) substituted phenyl$S(O)_n$ in which the substituents are X and Y,
(s) phenyl $C_{1-5}$ alkyl$S(O)_n$,
(t) $C_{1-5}$ alkyl$S(O)_n$,
(u) phenylaminoacyloxy,
(v) $C_{1-5}$ alkylaminoacyloxy,
(w) $C_{1-5}$ alkylacylamino,
(x) di(phenyl$C_{1-5}$alkyl)phosphonyl,
(y) di($C_{1-5}$alkyl)phosphinyl;
(5) $R_4$ together with the carbon atom to which it is attached represents a cyclopropane ring;

$R_5$ and $R_6$ independently are H, OH, $OR_7$ or $R_5$ and $R_6$ together with the carbon to which they are attached represent C=O or $R_5$ and $R_6$ together with the carbon to which they are attached represent a cyclopropane ring; provided that when $R_5$ is H, $R_6$ is OH or $OR_7$, and when $R_5$ is OH, $R_6$ is H, and when $R_5$ is $OR_7$, $R_6$ is H;

$R_7$ is

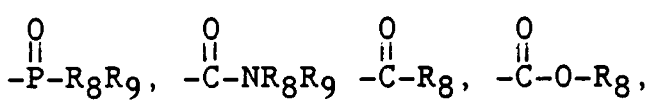

-phenyl$C_{1-3}$alkyl, $C_{1-5}$alkyl;

$R_8$ and $R_9$ independently are H, $C_{1-3}$alkyl, phenyl$C_{1-3}$alkyl or aryl wherein aryl is phenyl naphthyl, pyridyl, furanyl, thienyl or phenyl, naphthyl, pyridyl, furanyl or thienyl substituted with a groups X and Y provided that when $R_7$; is

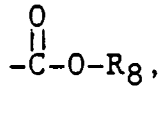

$R_8$ is not H and when $R_7$ is

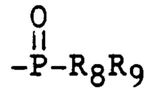

neither $R_8$ nor $R_9$ is H;

X and Y are independently selected from:

a) OH,
b) F,
c) trifluoromethyl,
d) $C_{1-3}$alkoxy,
e) hydrogen;
f) $C_{1-5}$alkyl.

In one subclass are the compounds of formulae (I) and (II) wherein:

$R_1$ is $C_{1-10}$alkyl; and

$R_4$ is $CH_3$, H, or $CH_2$phenyl.

Illustrative of this subclass are those compounds of formulae (I) and (II) wherein;

$R_7$ is

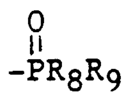, 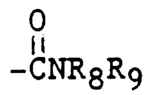 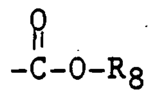,

$C_{1-5}$alkyl or phenyl$C_{1-3}$alkyl;

$R_8$ and $R_9$ independently are H, $C_{1-3}$alkyl, phenyl $C_{1-3}$alkyl or aryl wherein aryl is phenyl or naphthyl or phenyl or naphthyl substituted with X;

Further illustrating this subclass are those compounds wherein:

$R_1$ is 2-butyl or 2-methyl-2-butyl;

$R_4$ is $CH_3$.

Exemplifying this subclass are the following compounds:

6(R)-[2-[8(S)-(2,2-dimethylbutyryloxy)-2(S)-methyl-5(R)-hydroxy-6(R)-methyl-1,2,3,4,4a(R),5,6,7,8,8a(R)-decahydronaphthyl-1(S)]ethyl]-4(R)-hydroxy-3,4,5,6-tetrahydro-2H-pyran-2-one;

6(R)-[2-[8(S)-(2,2-dimethylbutyryloxy)-2(S)-methyl-5(R)-benzylaminocarbonyloxy-6(R)-methyl-1,2,3,4,4a(R)-,5,6,7,8,8a(R)-decahydronaphthyl-1(S)]ethyl]-4(R)-hydroxy-3,4,5,6-tetrahydro-2H-pyran-2-one;

6(R)-[2-[8(S)-(2,2-dimethylbutyryloxy)-2(S)-methyl-5(R)-diphenylphosphinyloxy-6(R)-methyl-1,2,3,4,4a(R)-,5,6,7,8,8a(R)-decahydronaphthyl-1(S)]ethyl]-4(R)-hydroxy-3,4,5,6-tetrahydro-2H-pyran-2-one;

6(R)-[2-[8(S)-(2,2-dimethylbutyryloxy)-2(S)-methyl-5(R)-(1-phenylethyl-2-oxy)-6(R)-methyl-1,2,3,4,4a(R)-,5,6,7,8,8a(R)-decahydronaphthyl-1(S)]ethyl]-4(R)-hydroxy-3,4,5,6-tetrahydro-2H-pyran-2-one;

6(R)-[2-[8(S)-(2,2-dimethylbutyryloxy)-2(S)-methyl-5(R)-dibenzylaminocarbonyloxy-6(R)-methyl-1,2,3,4,4a(R)-,5,6,7,8,8a(R)-decahydronaphthyl-1(S)]ethyl]-4(R)-hydroxy-3,4,5,6-tetrahydro-2H-pyran-2-one;

6(R)-[2-[8(S)-(2,2-dimethylbutyryloxy)-2(S)-methyl-5(R)-hydroxy-6(S)-methyl-1,2,3,4,4a(R),5,6,7,8,8a(R)-decahydronaphthyl-1(S)]ethyl]-4(R)-hydroxy-3,4,5,6-tetrahydro-2H-pyran-2-one;

6(R)-[2-[8(S)-(2,2-dimethylbutyryloxy)-2(S)-methyl-5(S)-hydroxy-6(S)-methyl-1,2,3,4,4a(R),5,6,7,8,8a(R)-decahydronaphthyl-1(S)]ethyl]-4(R)-hydroxy-3,4,5,6-tetrahydro-2H-pyran-2-one;

6(R)-{2-{8(S)-(2,2-dimethylbutyryloxy)-2(S)-methyl-5-oxo-6(R)-methyl-1,2,3,4,4a(R),6,7,8,8a(R)-nonahydronaphthyl-1(S)}ethyl}-4(R)-hydroxy-3,4,5,6-tetrahydro-2H-pyran-2-one;

6(R)-{2-{8(S)-(2,2-dimethylbutyryloxy)-2(S)-methyl-5-oxo-6(S)-methyl-1,2,3,4,4a(R),6,7,8,8a(R)-nonahydronaphthyl-1(S)}ethyl}-4(R)-hydroxy-3,4,5,6-tetrahydro-2H-pyran-2-one;

6(R)-[2-[8(S)-(2,2-dimethylbutyryloxy)-2(S)-methyl-5(R)-hydroxy-6(R)-methyl-1,2,4a(R),5,6,7,8,8a(R)-octahydronaphthyl-1(S)]ethyl]-4(R)-hydroxy-3,4,5,6-tetrahydro-2H-pyran-2-one; and the corresponding ring opened dihydroxy acids and esters thereof.

In a second subclass are the compounds of formula (I) and formula (II) wherein:

$R_1$ is $C_{1-10}$alkyl; and

$R_4$ is $CH_2OH$, or phenylacyloxymethyl.

Illustrative of this subclass are those compounds of formulae (I) and (II) wherein;

$R_7$ is

$$-\overset{O}{\overset{\|}{P}}R_8R_9, \quad -\overset{O}{\overset{\|}{C}}NR_8R_9,$$

$C_{1-5}$alkyl or phenyl$C_{1-3}$alkyl;

$R_8$ and $R_9$ independently are H, $C_{1-3}$-alkyl, phenyl$C_{1-3}$alkyl, or aryl wherein aryl is phenyl or naphthyl or phenyl or naphthyl substituted with X;

Further illustrating this subclass are those compounds wherein:

$R_1$ is 2-butyl or 2-methyl-2-butyl;

$R_4$ is $CH_2OH$.

Exemplifying this subclass are the following compounds:

6(R)-[2-[8(S)-(2,2-dimethylbutyryloxy)-2(S)-methyl-5(R)-hydroxy-6(R)-hydroxymethyl-1,2,3,4,4a(R),5,6,7,8,8a-(R)-decahydronaphthyl-1(S)]ethyl]-4(R)-hydroxy-3,4,5,6-tetrahydro-2H-pyran-2-one;

6(R)-[2-[8(S)-(2,2-dimethylbutyryloxy)-2(S)-methyl-5(R)-benzylaminocarbonyloxy-6(R)-hydroxymethyl-1,2,3,4,4a(R),5,6,7,8,8a(R)-decahydronaphthyl-1(S)]ethyl]-4(R)-hydroxy-3,4,5,6-tetrahydro-2H-pyran-2-one;

6(R)-[2-[8(S)-(2,2-dimethylbutyryloxy)-2(S)-methyl-5(R)-diphenylphosphinyloxy-6(R)-hydroxymethyl-1,2,3,4,4a(R),5,6,7,8,8a(R)-decahydronaphthyl-1(S)]ethyl]-4(R)-hydroxy-3,4,5,6-tetrahydro-2H-pyran-2-one;

6(R)-[2-[8(S)-(2,2-dimethylbutyryloxy)-2(S)-methyl-5(R)-(1-phenylethyl-2-oxy)-6(R)-hydroxymethyl-1,2,3,4,4a-(R),5,6,7,8,8a(R)-decahydronaphthyl-1(S)]ethyl]-4(R)-hydroxy-3,4,5,6-tetrahydro-2H-pyran-2-one;

6(R)-[2-[8(S)-(2,2-dimethylbutyryloxy)-2(S)-methyl-5(R)-dibenzylaminocarbonyloxy-6(R)-hydroxymethyl-1,2,3,4,4a(R),5,6,7,8,8a(R)-decahydronaphthyl-1(S)]ethyl]-4(R)-hydroxy-3,4,5,6-tetrahydro-2H-pyran-2-one;

6(R)-[2-[8(S)-(2,2-dimethylbutyryloxy)-2(S)-methyl-5(R)-hydroxy-6(S)-hydroxymethyl-1,2,3,4,4a(R),5,6,7,8,8a-(R)-decahydronaphthyl-1(S)]ethyl]-4(R)-hydroxy-3,4,5,6-tetrahydro-2H-pyran-2-one;

6(R)-[2-[8(S)-(2,2-dimethylbutyryloxy)-2(S)-methyl-5(S)-hydroxy-6(S)-hydroxymethyl-1,2,3,4,4a(R),5,6,7,8,8a-(R)-decahydronaphthyl-1(S)]ethyl]-4(R)-hydroxy-3,4,5,6-tetrahydro-2H-pyran-2-one; and the corresponding ring opened dihydroxy acids and esters thereof.

The compounds of formula (I) are prepared from lovastatin or mevastatin or a 6-desmethyl-6-hydroxymethyl or 8-acyloxy analog thereof, following the outline in Schemes 1 and 2.

SCHEME 1

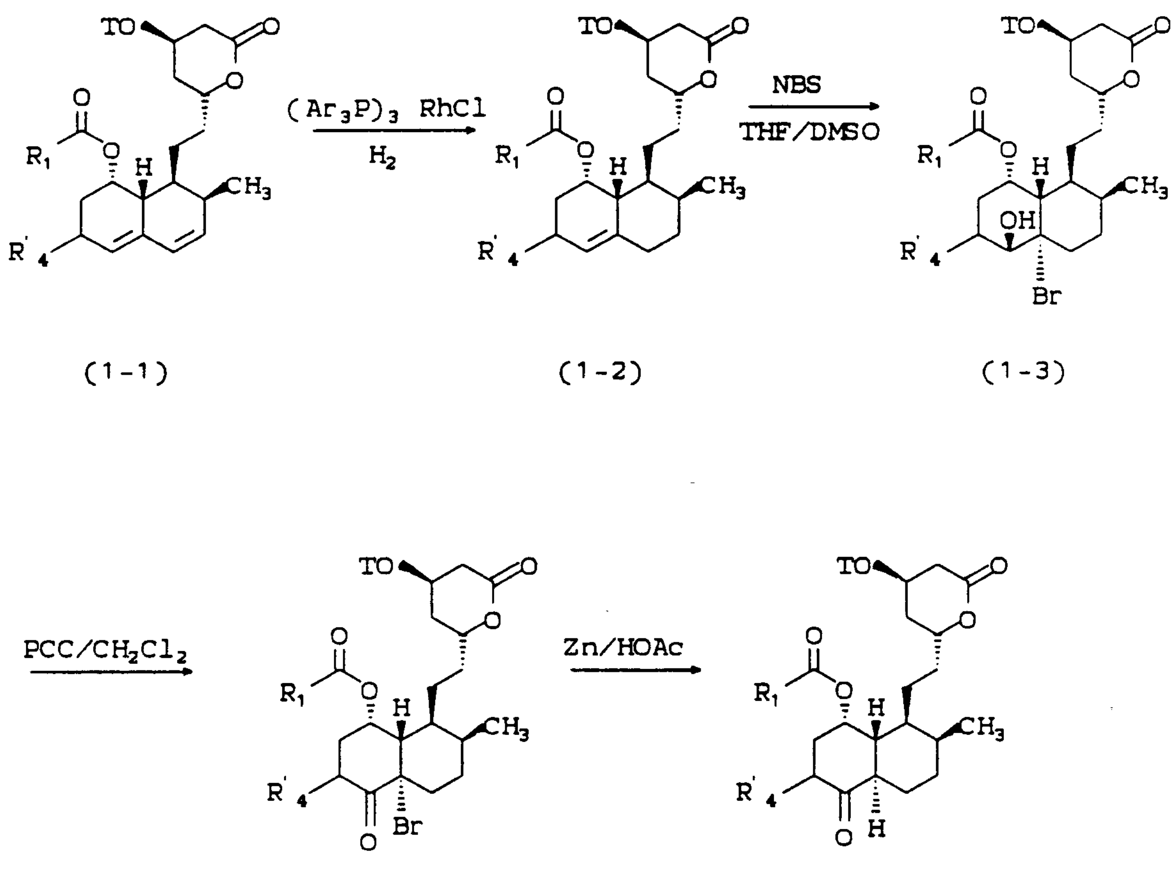


(1-1) (1-2) (1-3)

(1-4) (1-5)

SCHEME 1 (continued)

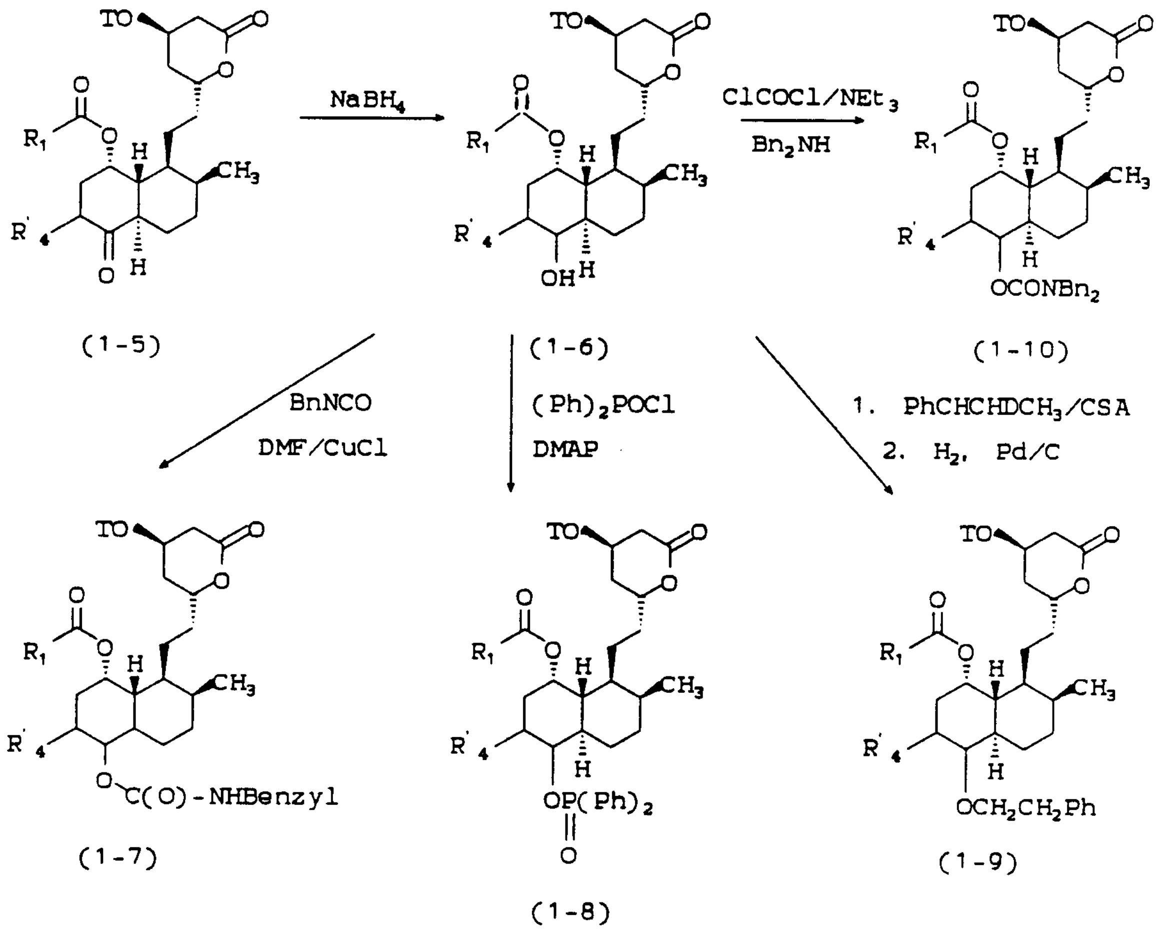


(1-5) (1-6) (1-10)

(1-7) (1-8) (1-9)

$R_4'$ = $CH_3$ or $CH_2OSi(Me)_2C_4H_9$-t, or H, T is a hydroxy protecting group such as tert-butyldimethylsilyl, tert-butyldiphenylsilyl, trimethylsilyl, triethylsilyl, triisopropylsilyl or tetrahydropyranyl.

Compound (1-2) is prepared from lovastatin by a reduction of the 3,4 double bond following the procedure detailed in EP 306210 (equivalent to US serial no. 092,804, filed September 3, 1987). The lactone hydroxyl may be protected or unprotected during this reduction. Where $R_4$ is 6-hydroxymethyl or a protected hydroxymethyl, the conversion of 6-methyl to 6-hydroxymethyl can be accomplished following the procedure in S.N. 254,525, filed October 6, 1988. The hydroxyl group in the lactone ring and at the 6-position of the polyhydronaphthyl ring may be protected (TO) using a silyl protecting group such as tert-butyldimethylsilyl, following the procedure in U.S. Patent 4,444,784. Where the acyl moiety is other than 2-methylbutyryl the acyl group of lovastatin may be hydrolyzed and the hydroxyl group reesterified with an appropriate alkanoyl halide following the procedure in U.S. Patent 4,444,784. The alkanoyl halide can be formed by standard transformations such as substitution with an alkyl moiety or other appropriate electrophile at an acidic C-H site on an available starting material.

The monoene (1-2) is converted to the bromohydrin (1-3) using NBS(N-bromosuccimide)/THF/DMSO. The bromohydrin (1-3) is oxidized to the intermediate bromoketone (1-4) with pyridinium chlorochromate (PCC) followed by reductive debromination in THF/acetic acid in the presence of zinc to afford ketone (1-5). Reduction of the ketone (1-5) with $NaBH_4$ in THF/$H_2O$ gave the epimeric 5-hydroxy derivatives (1-6). This reduction may be carried out on the hydroxyl protected ketone (1-5) or the unprotected ketone where T is H, preferred route is on the protected ketone where T is not H. The benzyl urethane (1-7) is prepared by treating the alcohol (1-6) with benzyl isocyanate in DMF in the presence of CuCl. The alcohol (1-6) can also be treated with $\beta$-methoxy styrene in $CH_2Cl_2$ in the presence of camphorsulfonic acid (CSA) to yield a Z-enol ether which is reduced with $H_2$ on 10% Pd/C in ethyl acetate to afford the phenethyl ether (1-9). The

phosphinate (1-8) is prepared from the alcohol (1-6) by treatment with diphenylphosphinic chloride and 4-dimethylaminopyridine (DMAP) in $CH_2Cl_2$. Dibenzylurethanes (1-10) are formed from the initial treatment of alcohol (1-6) with phosgene followed by reaction with dibenzylamine. The 5-alcohol moiety of compound (1-6) can also be converted to the ester, carbonate and ether functionalities by standard chemical transformations.

The 6-hydroxymethyl moiety is converted to a 6-iodomethyl moiety by iodination of the hydroxyl (e.g. iodine, triphenylphosphine, imidazole) followed by substitution or radical mediated coupling with an alkyl or heteroatom moiety which results in the elaboration of $CH_2I$ to $R_4$. One example of such methodology is the cross-coupling reaction between an alkyl halide and an organometallic reagent (e.g. alkyl iodides with lithium dialkyl copper-Posner, Org. React. 22, 253-400 (1975)).

Copending U.S. Patent Application S.N. 254,525 filed October 6, 1988, discloses a method of preparing the 6-α-desmethyl 6-β-methyl lovastatin derivative which can be employed as a starting material in the above scheme. Alternatively removal of the silyl protecting T of the 6-α-methyl ketone (1-5) followed by treatment with 1,8-diazabicyclo [5,4,0] undec-7-ene (DBU) results in the 6-β-methyl ketone (1-5) which after reprotection of the lactone hydroxy group and treatment with $NaBH_4$ gives a mixture of the 6-β-methyl-5(S)-hydroxy compound (1-6) and the 6-β-methyl-5(R)-hydroxy compound (1-6).

The methodology of Scheme 2 may be employed where the compounds of Formula (I) contain a double bond in the 3,4 position.

<u>SCHEME 2</u>

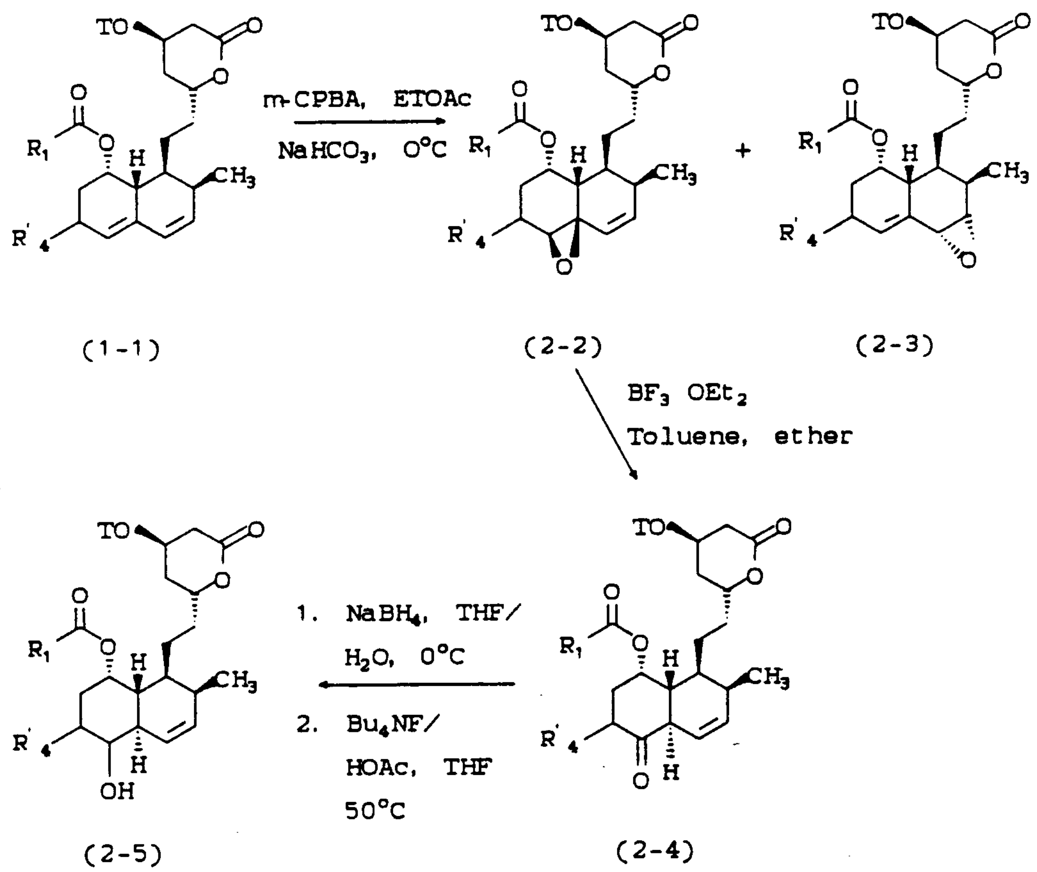


(1-1) (2-2) (2-3)

(2-5) (2-4)

The hydroxyl protected diene (1-1) is converted to epoxides (2-2) and (2-3) by treatment with m-chloroperoxybenzoic acid at about 0 ° C. Epoxide (2-2) is then treated with boron trifluoride etherate to form the enone (2-4). The ketone moiety is reduced with $NaBH_4$ and the lactone hydroxyl deprotected using tetrabutylammonium fluoride to form alcohol (2-5). As described above, the reduction with $NABH_4$ may be carried out on the protected or unprotected (T = H) ketone. The 5-alcohol moiety of compound (2-5) can

be further treated to form the $OR_7$ moieties as described in Scheme 1.

Alternatively the double bond may be inserted following the methodology of Scheme 3.

The bromoketone (1-4), formed as an intermediate in Scheme 1, is isolated and dehydrobrominated with pyridine at about 60 ° C to form the eneone (3-2). Thiophenol is added to the olefin of (3-2) to form compound (3-3), which then undergoes oxidative elimination with m-chloroperoxybenzoic acid to form compound (3-4). Ketone (3-4) can be deprotected (3-5) and reduced to alcohol (3-6) which can by standard chemistry be converted to any of the $OR_7$ moieties described above.

Where the reaction conditions of the above noted chemical transformations would be deleterious to the substituents in the 8-acyloxy moiety, the acetoxy group can be employed as a protecting group which after the elaboration of the 5-position can be removed by hydrolysis to give the 8-hydroxy derivative which then can be acylated according to the general procedures described in U.S. Patent 4,661,483.

SCHEME 3

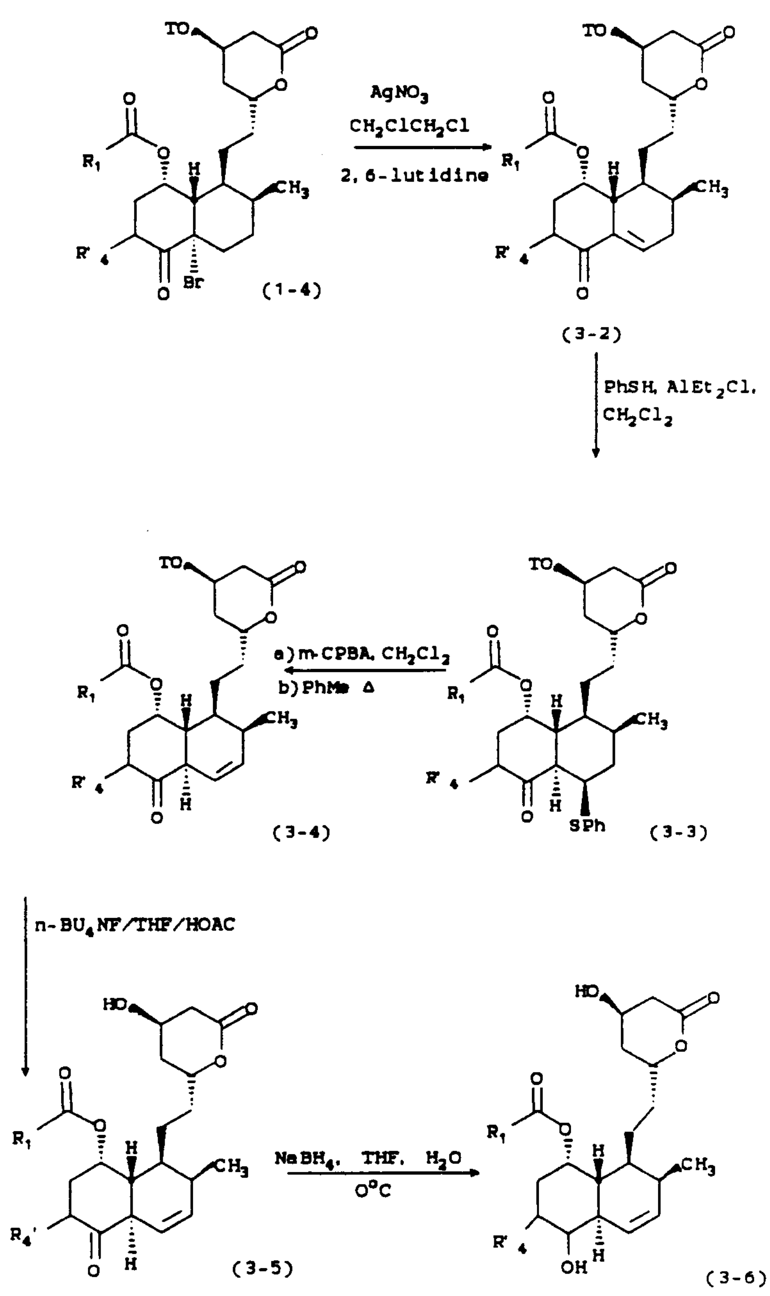


T is a hydroxyl protecting group such as tert-butyldimethylsilyl

Where the product formed by the above described synthetic pathways is not the desired form of that compound, then that product may be subjected to one or more further reactions such as hydrolysis, disilylation, salification, esterification, acylation, ammonolysis or lactonization by conventional methods.

Preferred metal salts are salts with alkali metals, such as sodium or potassium, salts with alkaline earth metals, such as calcium, or salts with other metals such as magnesium, aluminum, iron, zinc, copper, nickel

or cobalt, of which the alkali metal, alkaline earth metal, magnesium and aluminum salts are preferred, the sodium, calcium and aluminum salts being most preferred.

Preferred amino acids to form amino acid salts are basic amino acids, such as arginine, lysine, α,β-diaiminobutyric acid or ornithine.

Preferred amines to form amine salts include t-octylamine, dibenzylamine, ethylenediamine, morpholine, and tris(hydroxymethyl)aminomethane. Also preferred is ammonia to form the ammonium salt.

Esters are preferably the alkyl esters, such as the methyl, ethyl, propyl, isopropyl, butyl, isobutyl, or pentyl esters, of which the methyl ester is preferred. However, other esters such as phenyl$C_{1-5}$alkyl, dimethylamino-$C_{1-5}$alkyl, or acetylamino$C_{1-5}$alkyl may be employed if desired.

Metal salts of the carboxylic acids of formula (II) may be obtained by contacting a hydroxide, carbonate or similar solvent with the carboxylic acid of formula (II). The aqueous solvent employed is preferably water, or it may be a mixture of water with an organic solvent, preferably an alcohol (such as methanol or ethanol), a ketone (such as acetone), an aliphatic hydrocarbon (such as hexane) or an ester (such as ethyl acetate). It is preferred to use a mixture of a hydrophilic organic solvent with water. Such reactions are normally conducted at ambient temperature but they may, if desired, be conducted with heating or cooling.

Amine salts of the carboxylic acids of formula (II) may be obtained by contacting an amine in an aqueous solvent with the carboxylic acid of formula (II). Suitable aqueous solvents include water and mixtures of water with alcohols (such as methanol or ethanol), ethers (such as diethyl ether and tetrahydrofuran), nitriles (such as acetonitrile) or ketones (such as acetone); it is preferred to use aqueous acetone as the solvent for this reaction. The reaction is preferably carried out at a temperature of ambient or below, more preferably a temperature of from 5° to 10°C. The reaction immediately goes to completion. Alternatively, a metal salt of the carboxylic acid of formula (II) (which may have been obtained as described above) can be dissolved in an aqueous solvent, after which a mineral acid salt (for example the hydrochloride) of the desired amine is added, employing the same reaction conditions as when the amine itself is reacted with the carboxylic acid of formula (II) and the desired product is then obtained by metathesis.

Amino acid salts of the carboxylic acids of formula (II) may be obtained by contacting an amino acid in aqueous solution with the carboxylic acid of formula (II). Suitable aqueous solvents include water and mixtures of water with alcohols (such as methanol or ethanol) or ethers (such as tetrahydrofuran).

Esters, preferably alkyl esters, of the carboxylic acids of formula (II) may be obtained by contacting the carboxylic acid of formula (II) with an appropriate alcohol, preferably in the presence of an acid catalyst, for example a mineral acid (such as hydrochloric acid or sulphuric acid), a Lewis acid (for example boron trifluoride) or an acidic ion exchange resin. The solvent employed for this reaction is not critical, provided that it does not adversely affect the reaction; suitable solvents include the alcohol itself, benzene, chloroform, ethers and the like. Alternatively, the desired product may be obtained by contacting the carboxylic acid of formula (II) with a diazoalkane, in which the alkane moiety may be substituted or unsubstituted. This reaction is usually effected by contacting the acid with an ethereal solution of the diazoalkane. As a further alternative, the ester may be obtained by contacting a metal salt of the carboxylic acid of formula (II) with a halide, preferably an alkyl halide, in a suitable solvent; preferred solvents include dimethylformamide, tetrahydrofuran, dimethylsulfoxide and acetone. Finally, esters may also be obtained from the lactone of formula (I) by reaction with an appropriate alkoxide in an absolute alkanol. All of the reactions for producing esters are preferably effected at about ambient temperature, but, if required by the nature of the reaction system, the reactions may be conducted with heating or cooling.

Lactones of the carboxylic acids of formula (I) may be obtained by lactonizing the carboxylic acids of formula (II) under ordinary conditions known to one skilled in the art.

The intrinsic HMG-CoA reductase inhibition activity of the claimed compounds is measured in the in vitro protocol published in J. Med. Chem., 28, p. 347-358 (1985).

For estimation of relative inhibitory potencies, compactin (i.e., mevastatin) was assigned a value of 100 and the $IC_{50}$ value of the test compound was compared with that of compactin determined simultaneously in the published in vitro protocol.

Representative of the intrinsic HMG-CoA reductase inhibitory activities of the claimed compounds are the following relative potencies for compounds of formula (I):

| Compound | Relative Potency |
|---|---|
| 6(R)-[2-[8(S)-(2,2-dimethylbutyryloxy)-2(S)-methyl-5(R)-diphenylphosphinyloxy-6(R)-methyl-1,2,3,4,4a(R),5,6,7,8,8a(R)-decahydronaphthyl-1(S)]ethyl]-4(R)-hydroxy-3,4,5,6-tetrahydro-2H-pyran-2-one | 300 |

| Compound | Relative Potency |
|---|---|
| 6(R)-[2-[8(S)-(2,2-dimethylbutyryloxy)-2(S)-methyl-5(R)-benzylaminocarbonyloxy-6(R)-methyl-1,2,3,4,4a(R),5,6,7,8,8a(R)-decahydronaphthyl-1(S)]ethyl]-4(R)-hydroxy-3,4,5,6-tetrahydro-2H-pyran-2-one | 360 |
| 6(R)-[2-[8(S)-(2,2-dimethylbutyryloxy)-2(S)-methyl-5(R)-(1-phenylethyl-2-oxy)-6(R)-methyl-1,2,3,4,4a(R),5,6,7,8,8a(R)-decahydronaphthyl-1(S)]ethyl]-4(R)-hydroxy-3,4,5,6-tetrahydro-2H-pyran-2-one | 300 |
| 6(R)-[2-[8(S)-(2,2-dimethylbutyryloxy)-2(S)-methyl-5(R)-dibenzylaminocarbonyloxy-6(R)-methyl-1,2,3,4,4a(R),5,6,7,8,8a(R)-decahydronaphthyl-1(S)]ethyl]-4(R)-hydroxy-3,4,5,6-tetrahydro-2H-pyran-2-one | 94 |
| 6(R)-[2-[8(S)-(2,2-dimethylbutyryloxy)-2(S)-methyl-5,(R)-hydroxy-6(R)-methyl-1,2,3,4,4a(R),5,6,7,8,8a(R)-decahydronaphthyl-1(S)]ethyl]-4(R)-hydroxy-3,4,5,6-tetrahydro-2H-pyran-2-one | 50 |
| 6(R)-[2-[8(S)-(2,2-dimethylbutyryloxy)-2(S)-methyl-5(R)-hydroxy-6(S)-methyl-1,2,3,4,4a(R),5,6,7,8,8a(R)-decahydronaphthyl-1(S)]ethyl]-4(R)-hydroxy-3,4,5,6-tetrahydro-2H-pyran-2-one | 99.6 |

| Compound | Relative Potency |
|---|---|
| 6(R)-[2-[8(S)-(2,2-dimethylbutyryloxy)-2(S)-methyl-5(S)-hydroxy-6(S)-methyl-1,2,3,4,4a(R),5,6,7,8,8a(R)-decahydronaphthyl-1(S)]ethyl]-4(R)-hydroxy-3,4,5,6-tetrahydro-2H-pyran-2-one | 11.1 |
| 6(R)-[2-[8(S)-(2,2-dimethylbutyryloxy)-2(S)-methyl-5-oxo-6(R)-methyl-1,2,3,4,4a(R),6,7,8,8a(R)-nonahydronaphthyl-1(S)]ethyl]-4(R)-hydroxy-3,4,5,6-tetrahydro-2H-pyran-2-one | 125 |
| 6(R)-[2-[8(S)-(2,2-dimethylbutyryloxy)-2(S)-methyl-5-(R)-hydroxy-6(R)-methyl-1,2,4a(R),5,6,7,8,8a(R)-octahydronaphthyl-1(S)]ethyl]-4(R)-hydroxy-3,4,5,6-tetrahydro-2H-pyran-2-one. | 100 |

The compounds of this invention are useful as antihypercholesterolemic agents for the treatment of arteriosclerosis, hyperlipidemia, familial hypercholesterolemia and like diseases in humans. They may be administered orally or parenterally in the form of a capsule, a tablet, an injectable preparation or the like. It is usually desirable to use the oral route. Doses may be varied, depending on the age, severity, body weight and other conditions of human patients but daily dosage for adults is within the range of from about 10 mg to 2000 mg (preferably 10 to 100 mg) which may be given in two to four divided doses.

The compounds of this invention may also be coadministered with pharmaceutically acceptable nontoxic cationic polymers capable of binding bile acids in a non-reabsorbable form in the gastrointestinal tract. Examples of such polymers include cholestyramine, coletipol and poly[methyl-(3-trimethylaminopropyl)imino-trimethylene dihalide]. The relative amounts of the compounds of this invention and these polymers is between 1:100 and 1:15,000.

Included within the scope of this invention is the method of treating arteriosclerosis, familial hypercholesterolemia or hyperlipidemia which comprises administering to a subject in need of such treatment a nontoxic, therapeutically-effective amount of the compounds of formulae (I) or (II) or pharmaceutical compositions thereof.

Also included within the scope of the present invention is the process for the formation of the 5-hydroxy compound wherein <u>a</u> is a single or a double bond. Further included within the scope of this invention are the intermediate compounds (1-4) and (3-2).

The following examples illustrate the preparation of the compounds of the formulae (I) and (II) and their incorporation into pharmaceutical compositions and as such are not to be considered as limiting the invention set forth in the claims appended hereto:

EXAMPLE 1

Preparation of 6(R)-[2-[8(S)-(2,2-dimethylbutyryloxy)-2(S)-methyl-5(R)-hydroxy-6(R)-methyl-1,2,3,4,4a(R)-,5,6,7,8,8a(R)-decahydronaphthyl-1(S)]ethyl]-4(R)-hydroxy-3,4,5,6-tetrahydro-2H-pyran-2-one (I-6)

Step 1: Preparation of 6(R)-[2-(8(S)-(2,2-dimethylbutyryloxy)-2(S)-methyl-6(R)-methyl-1,2,3,4,6,7,8,8a(S)-octahydronaphthyl-1(S)]ethyl]-4(R)-hydroxy-3,4,5,6-tetrahydro-2H-pyran-2-one (1).

Nitrogen was bubbled through a solution of 50% toluene in absolute ethanol (300 mL) for 5 minutes. Wilkinson's catalyst (5.0 g, 33%/wt.) was added to the solvent and the mixture reduced at room temperature under 50 psi $H_2$ for 1 hour. Simvastatin (15 g, 36 mmol) was added and the resulting pale yellow solution reduced at room temperature under $H_2$ (60 psi) for 40 hours. The mixture was concentrated and the residue heated in toluene (700 mL) at 60°C in the presence of thiourea (5.0 g, 64 mmol) for 1.5 hours. The mixture was cooled to 0°C (ice bath), filtered, and concentrated. The residue was diluted with 50% EtOAc/hexane and passed through a pad of silica (~250 cc) to give 2 as a beige solid; mp = 128-129°C (ethyl acetate/hexane); TLC $R_f$ = 0.65 (EtOAc); $^1$H NMR*($CDCl_3$) δ 5.36 (bs, 1H), 5.30 (m,1H), 4.58 (m,1H), 4.33 (m,1H), 2.68 (dd,J = 17 and 5Hz,1H), 2.68 (m,1H), 2.59 (dd, J = 17 and 4Hz,1H), 2.30-1.20 (m), 1.13 (s,3H), 1.12 (s,3H), 1.05 (d, J = 7Hz,3H), 0.87 (d, J = 7Hz,3H), 0.82 (t, J = 7Hz,3H).

Step 2: Preparation of 6(R)-[2-[8-(S)-(2,2-dimethylbutyryloxy)-2(S),6(R)-dimethyl-1,2,3,4,5,7,8,8a(R)-octahydronaphthyl-1(S)]ethyl]-4(R)-tert-butyldimethylsilyloxy-3,4,5,6-tetrahydro-2H-pyran-2-one (2)

To a stirred solution of alcohol 1 (5.0 g, 11.9 mmol), imidazole (1.8 g, 26.2 mmol) and dry DMF (30 mL) at 25°C was added tert-butyldimethylsilyl chloride (2.0 g, 13.0 mmol). After 4 hours the reaction mixture was diluted with petroleum ether, washed with $H_2O$ (2X) and brine, dried ($MgSO_4$), and concentrated to give the silyl ether 2 as a colorless oil ; TLC Rf = 0,70 (50% ether/pet. ether); $^1$H NMR ($CDCl_3$) δ 5.39 (m, 1H,) 5.28 (m, 1H), 4.57 (m, 1H), 4.27 (m, 1H), 2.57 (m, 2H), 2.20-1.20 (m), 1.15 (s, 3H), 1.14 (s, 3H), 1.07 (d, J = 7Hz, 3H), 0.86 (d, J = 7Hz, 3H), 0.82 (t, J = 7Hz, 3H).

Step 3: Preparation of 6(R)-[2-(8(S)-(2,2-dimethylbutyryloxy)-2(S)-methyl-4a(S)-bromo-5(S)-hydroxy-6(R)-methyl-1,2,3,4,5,6,7,8,8a(R)-nonahydronaphthyl-1(S)]-ethyl]-4(R)-tert-butyldimethylsilyloxy-3,4,5,6-tetrahydro-2H-pyran-2-one (3)

To a stirred solution of 6(R)-[2-[8(S)-(2,2-dimethylbutyryloxy)-2(S)-methyl-6(R)-methyl-1,2,3,46,7,8,8a(R)-octahydronaphthyl-1(S)]ethyl]-4(R)-tert-butyldimethylsilyloxy-3,4,5,6-tetrahydro-2H-pyran-2-one (95 mg, 0.23 mmol), DMSO (1.0 mL), THF (0.5 mL), and $H_2O$ (12 µL, 0.7 mmol) at 5°C was added N-bromosuccinimide (NBS) (61 mg, 0.33 mmol). After 1 hour the yellow reaction mixture was diluted with ether, washed with $H_2O$, saturated $NaHCO_3$ and brine, dried ($MgSO_4$), and concentrated. Flash chromatography (silica, 30% EtOAc/hexane) furnished the bromohydrin as a colorless oil.
$^1$H NMR ($CDCl_3$) δ 5.08(m, 1H), 4.54(m, 1H), 4.26(m, 1H), 4.13(d, J=3 Hz,1H), 2.63-2.48(m, 2H), 2.35-1.1-(m), 1.31(d, J=6 Hz, 3H), 1.13(s, 3H), 1.12(s, 3H), 0.87(s, 9H), 0.8(m, 6H) 0.05(s, 3H), 0.04(s,3H).

Step 4: Preparation of 6(R)-[2-[8(S)-(2,2-dimethylbutyryloxy)-2(s)-methyl-4a(S)-bromo-5-oxo-6(R)-methyl-1,2,3,4,6,7,8,8a(R)-octahydronaphthyl-1(s)]ethyl]-4(R)-tert-butyldimethylsilyloxy-3,4,5,6-tetrahydro-2H-pyran-2-one(4)

To a stirred mixture of compound 3 (2.4 g, 3.8 mmol), 4A sieves (2.5 g), and dry $CH_2Cl_2$ (19 ml) at 0°C was added pyridinium chlorochromate (PCC) (3.2 g, 14.8 mmol). After stirring for 30 minutes, the icebath was removed with continued stirring for 30 minutes. The reaction mixture was diluted with ether and filtered through a celite pad into a filtration flask containing acetic acid (0.8 mL, 14.0 mmol). Concentration at 10°C gave the crude bromoketone 4. Flash chromatography (silica, 15% EtOAc/hexanes) gave compound 4 as a solid (m.p. 85-87°C).
'H NMR ($CDCl_3$) δ 5.24(m,1H), 4.60(m,1H), 4.32 (m,1H), 2.75(m,1H), 2.62(m,2H), 2.40-1.20(m), 1.24-(d,J=7Hz,3H), 1.21(s,3H), 1.19(s,3H),0.91(s,9H), 0.89(m,6H), 0.11(s,3H),0.10(s,3H).

*NMR spectra were measured on a Varian XL-300 spectrometer.

Step 5: Preparation of 6(R)-[2-[8(S)-(2,2-dimethylbutyryloxy)-2(S)-methyl-5-oxo-6(R)-methyl-1,2,3,4,4a(R)-,6,7,8,8a(R)-nonahydronaphthyl-1(S)lethyl]-4(R)-tert-butyldimethylsilyloxy-3,4,5,6-tetrahydro-2H-pyran-2-one (5)

The crude bromoketone 4 (2.6 g, 3.8 mmol) was dissolved in THF/HOAc (38 mL) followed by treatment with zinc (0.74 g, 11.4 mmol) at ambient temperature. After 1.0 hour of vigorous stirring, the reaction mixture was diluted with ether and the excess zinc removed by filtration. The filtrate was washed with $H_2O$ and brine, dried ($MgSO_4$), and concentrated. Flash chromatography (silica, 15% EtOAc/hexanes) gave compound 5 as a solid (m.p. 147-148 °C).
$^1H$ NMR ($CDCl_3$) $\delta$ 5.31(m, 1H), 4.60(m, 1H), 4.29(m,1H), 2.58(m,2H), 2.24-1.20(M), 1.24(d, J = 7Hz,3H, 1.88-(s, 3H), 1.17(s,3H), 0.89(s,9H), 0.87(d, J = 7Hz, 3H), 0.83(t, J = 7Hz,3H), 0.06(s, 6H).

Step 6: Preparation of 6(R)-[2-[8(S)-(2,2-dimethylbutyryloxy)-2(S)-methyl-5(R)-hydroxy-6(R)-methyl-1,2,3,4,4a(R),5,6,7,8,8a(R)-decahydronaphthyl-1(S)]ethyl]-4(R)-tert-butyldimethylsilyloxy-3,4,5,6-tetrahydro-2H-pyran-2-one (6)

To a stirred solution of compound 5 (320 mg, 0.58 mmol), THF (2.6 mL), and $H_2O$ (0.3 mL) at 0 °C was added $NaBH_4$ (66 mg, 1.7 mmol). After 35 minutes, the reaction mixture was diluted with ethyl acetate, washed with $H_2O$ (2X) and brine, dried ($MgSO_4$), and concentrated. Flash chromatography (silica, 20% ethyl acetate/hexane) gave compound 6 as a colorless oil.
$^1H$ NMR ($CDCl_3$) $\delta$ 5.06(m, 1H), 4.60(m, 1H), 4.14(m, 1H), 3.45(dd, J = 10 and 5Hz, 1H), 2.56(m, 2H), 2.15-1.15(m), 1.17(s, 3H), 1.16(s, 3H), 1.07(d, J = 7Hz, 3H), 0.88(s, 9H), 0.88(t, J = 7Hz, 3H), 0.86(d, J = 7Hz, 3H), 0.08(s, 3H), 0.08(s, 3H).

Step 7: Preparation of 6(R)-[2-[8(S)-(2,2-dimethylbutyryloxy)-2(S)-methyl-5(R)-hydroxy-6(R)-methyl-1,2,3,4,4a(R),6,7,8,8a(R)-decahydronaphthyl-1(S)]ethyl]-4(R)-hydroxy-3,4,5,6-tetrahydro-2H-pyran-2-one (I-6)

To a stirred solution of compound 6 (98 mg, 0.18 mmol), THF (530 µL), and HOAc (41 µL, 0.71 mmol) was added tetrabutylammonium fluoride (1M THF, 530 µL, 0.53 mmol) at ambient temperature. After 20 hours, the reaction mixture was diluted with ethyl acetate, washed with $H_2O$ and brine, dried ($MgSO_4$), and concentrated. Flash chromatography (silica, 60% EtOAc/hexane) gave compound (I-6) as a crystalline solid.
mp = 142-143 °C
$^1H$ NMR ($CDCl_3$) $\delta$ 5.05(m, 1H), 4.54(m, 1H), 4.31(m, 1H), 3.42(dd, J = 10 and 5Hz, 1H), 2.69(dd, J = 17 and 5Hz, 1H), 2.57(dd, J = 17 and 4Hz, 1H), 2.12-1.10(m), 1.17(s, 3H), 1.16(s, 3H), 1.06(d, J = 7Hz, 3H), 0.82(t, J = 7Hz, 3H), 0.79(d, J = 7Hz, 3H).

| Elemental Anal. $C_{25}H_{42}O_6 \cdot 0.5H_2O$: | | |
|---|---|---|
| Calc'd: | C, 67.08; | H, 9.68 |
| Found: | C, 66.84; | H, 9.31 |

EXAMPLE 2

Preparation of 6(R)-[2-[8(S)-(2,2-dimethylbutyryloxy)-2(S)-methyl-5(R)-benzylaminocarboxy-6(R)-methyl-1,23,4,4a(R),5,6,7,8,8a(R)-decahydronaphthyl-1(S)]ethyl]-4(R)-hydroxy-3,4,5,6-tetrahydro-2H-pyran-2-one (I-7)

Step 1: Preparation of 6(R)-[2-[8(S)-(2,2-dimethylbutyryloxy)-2(S)-methyl-4a(S)-bromo-5(S)-hydroxy-6(R)-methyl-1,2,3,4,5,6,7,8,8a(R)-nonahydronaphthyl-1(S)]ethyl]-4(R)-tert-butyldimethylsilyloxy-3,4,5,6-tetrahydro-2H-pyran-2-one (3)

To a stirred solution of 6(R)-[2-[8(S)-)2,2-dimethylbutyryloxy)-2(S)-methyl-6(R)-methyl-1,2,3,4,6,7,8,8a-(R)-octahydronaphthyl-1(S)]ethyl]-4(R)-tert-butyldimethylsilyloxy-3,4,5,6-tetrahydro-2H-pyran-2-one (95 mg, 0.23 mmol) DMSO (1.0 mL), THF (0.5 mL), and $H_2O$ (12 µL, 0.7 mmol) at 5 °C was added N-bromosuccinimide (NBS) (61 mg, 0.33 mmol). After 1 hour, the yellow reaction mixture was diluted with ether, washed with $H_2O$, saturated with $NaHCO_3$, and brine, dried ($MgSO_4$), and concentrated. Flash chromatography (silica, 30% EtOAc/hexane) furnished the bromohydrin as a colorless oil.

$^1H$ NMR ($CDCl_3$) δ 5.08(m, 1H), 4.54(m, 1H), 4.26(m, 1H), 4.13(d, J=3 Hz, 1H), 2.63-2.48(m, 2H), 2.35-1.1-(m), 1.31(d, J=6 Hz, 3H), 1.13(s, 3H), 1.12(s, 3H), 0.87(s, 9H), 0.8(m, 6H), 0.05(s, 3H), 0.04(s,3H).

Step 2: Preparation of 6(R)-[2-[8(S)-(2,2-dimethylbutyryloxy)-2(S)-methyl-5-oxo-6(R)-methyl-1,2,3,4,4a(R)-,6,7,8,8a(R)-nonahydronaphthyl-1(S)]ethyl]-4(R)-tert-butyldimethylsilyloxy-3,4,5,6-tetrahydro-2H-pyran-2-one (5)

To a stirred mixture of compound 3 (2.4 g, 3.8 mmol), 4A sieves (2.5 g), and dry $CH_2Cl_2$ (19 ml) at 0°C was added pyridinium chlorochromate (PCC) (3.2 g, 5.2 mmol). After stirring for 30 minutes, the icebath was removed with continued stirring for 30 minutes. The reaction mixture was diluted with ether and filtered through a celite pad into a filtration flask containing acetic acid (0.8 mL, 14.0 mmol). Concentration at 10°C gave the crude bromoketone which was reduced immediately. The crude bromoketone was dissolved in THF/HOAc (38 mL) followed by treatment with zinc (0.74 g, 11.4 mmol) at ambient temperature. After 1.0 hour of vigorous stirring, the reaction mixture was diluted with ether and the excess zinc removed by filtration. The filtrate was washed with $H_2O$ and brine, dried ($MgSO_4$), and concentrated. Flash chromatography (silica, 15% EtOAc/hexanes) gave compound 5 as a solid. (m.p. 147-148°C)
$^1H$ NMR ($CDCl_3$) δ 5.31(m, 1H), 4.60(m, 1H), 4.29(m, 1H), 2.58(m, 2H), 2.24-1.20(m), 1.24(d, J=7Hz, 3H), 1.88(s, 3H), 1.17(s, 3H), 0.89(s, 9H), 0.87(d, J=7Hz, 3H), 0.83(t, J=7Hz, 3H), 0.06(s, 6H).

Step 3: Preparation of 6(R)-[2-[8(S)-(2,2-dimethylbutyryloxy)-2(S)-methyl-5(R)-hydroxy-6(R)-methyl-1,2,3,4,4a(R),5,6,7,8,8a(R)-decahydronaphthyl-1(S)]ethyl]-4(R)-tert-butyldimethylsilyloxy-3,4,5,6-tetrahydro-2H-pyran-2-one (6)

To a stirred solution of compound 5 (320 mg, 0.58 mmol), THF (2.6 mL), and $H_2O$ (0.3 mL) at 0°C was added $NaBH_4$ (66 mg, 1.7 mmol). After 35 minutes, the reaction mixture was diluted with ethyl acetate, washed with $H_2O$ (2X) and brine, dried ($MgSO_4$), and concentrated. Flash chromatography (silica, 20% ethyl acetate/hexane) gave compound 6 as a colorless oil.
$^1H$ NMR ($CDCl_3$) δ 5.06(m, 1H), 4.60(m, 1H), 4.14(m, 1H), 3.45(dd, J=10 and 5Hz, 1H), 2.56(m, 2H), 2.15-1.15(m), 1.17(s, 3H), 1.16(s, 3H), 1.07(d, J=7Hz, 3H), 0.88(s, 9H), 0.88(t, J=7Hz, 3H), 0.86(d, J=7Hz, 3H), 0.08(s, 3H), 0.08(s, 3H).

Step 4: Preparation of 6(R)-[2-[8(S)-(2,2-dimethylbutyryloxy)-2(S)-methyl-5(R)-benzylaminocarbonyloxy-6(R)-methyl-1,2,3,4,4a(R),5,6,7,8,8a(R)-decahydronaphthyl-1(S)]ethyl]-4(R)-tert-butyldimethylsilyloxy-3,4,5,7-tetrahydro-2H-pyran-2-one (7)

To a mixture of compound 6 (227 mg, 0.41 mmol), degassed DMF (2.0 mL), and CuCl (41 mg, 0.41 mmol) at 25°C was added benzyl isocyanate (82 mg, 0.62 mmol). After 1 hour, the dark green mixture was diluted with ether, washed with $H_2O$ and brine, dried ($MgSO_4$), and concentrated. Flash chromatography (silica, 20% EtOAc/hexane) furnished compound 7 as a colorless oil.
$^1H$ NMR ($CDCl_3$) δ 7.30(m, 5H), 5.06(m, 1H), 4.93(m, 1H), 4.61(dd, J=10 and 5Hz, 1H), 4.37(d, J=6Hz, 2H), 4.25(m, 1H), 2.55(m, 2H), 2.27(m, 1H), 2.00-1.10(m), 1.14(s, 3H,), 1.13(s, 3H), 0.86(s, 9H), 0.80(m, 9H), 0.06-(s, 6H).

Step 5: Preparation of 6(R)-[2-[8(S)-(2,2-dimethylbutyryloxy)-2(S)-methyl-5(R)-benzylaminocarbonyloxy-6(R)-methyl-1,2,3,4,4a(R),5,6,7,8,8a(R)-decahydronaphthyl-1(S)]ethyl]-4(R)-hydroxy-3,4,5,6-tetrahydro-2H-pyran-2-one (1-7)

Utilizing the same procedure of Example 1, Step 7, the compound 7 (80 mg, 0.11 mmol) was converted to the desired compound (I-7) which was an amorphous solid.
$^1H$ NMR ($CDCl_3$) δ 7.30(m, 5H), 5.08(m, 1H), 5.02(t, J=6Hz, 1H), 4.59(dd, J=10 and 5Hz, 1H), 4.54(m, 1H), 4.34(d, J=6Hz, 2H), 4.30(m, 1H), 3.03(bs, 1H), 2.69(dd, J=18 and 5Hz, 1H), 2.58(dd, J=18 and 4Hz, 1H), 2.26(m, 1H), 2.00-1.10(m), 1.14(s, 3H), 1.13(s, 3H), 0.82(t, J=7Hz, 3H), 0.78(d, J=7Hz, 3H).

| Elemental Anal. $C_{33}H_{49}O_7N \cdot 1.5H_2O$ | | | |
|---|---|---|---|
| Calc'd: | C, 66.20; | H, 8.75 | N, 2.34 |
| Found: | C, 65.86; | H, 8.99 | N, 2.03 |

EXAMPLE 3

Preparation of 6(R)-[2-[8(S)-(2,2-dimethylbutyryloxy)-2(S)-methyl-5(R)-hydroxy-6(S)-methyl-1,2,3,4,4a(R)-,6,7,8,8a(R)-decahydronaphthyl-1(S)]ethyl]-4(R)-hydroxy-3,4,5,6-tetrahydro-2H-pyran-2-one (14)

and

6(R)-[2-[8(S)-(2,2-dimethylbutyryloxy)-2(S)-methyl-5(S)-hydroxy-6(S)-methyl-1,2,3,4,4a(R),6,7,8,8a(R)-decahydronaphthyl-1(S)]ethyl]-4(R)-hydroxy-3,4,5,6-tetrahydro-2H-pyran-2-one (15)

Example 1, Steps 1-5 were repeated but substituting tert-butyldiphenylsilyl as the hydroxyl protecting group.

Step 6: Preparation of 6(R)-[2-[8(S)-(2,2-dimethylbutyryloxy)-2(S)-methyl-5-oxo-6(R)-methyl-1,2,3,4,4a(R)-,6,7,8,8a(R)-nonahydronaphthyl-1(S)]ethyl]-4(R)-hydroxy-3,4,5,6-tetrahydro-2H-pyran-2-one (12)

To a stirred solution of 6(R)-[2-[8(S)-(2,2-dimethylbutyryloxy)-2(S)-methyl-5-oxo-6(R)-methyl-1,2,3,4,4a-(R),6,7,8,8a(R)-nonahydronaphthyl-1(S)]ethyl]-4(R)-tert-butyldiphenylsilyloxy-3,4,5,6-tetrahydro-2H-pyran-2-one (345 mg, 0.63 mmol) and $CH_2Cl_2$ (3.1 mL) at 0°C was added HF•pyridine (0.19 g). After 1 hour, the reaction mixture was diluted with ethyl acetate, washed carefully with saturated $NaHCO_3$ and brine, dried ($MgSO_4$), and concentrated. Flash chromatography (silica, 50% ethyl acetate/hexane) furnished compound 12 as a colorless solid.
mp = 159-160°C
$^1$H NMR ($CDCl_3$) δ 5.36(m, 1H), 4.63(m, 1H), 4.40(m, 1H), 2.78(dd, J=18 and 5Hz, 1H), 2.60(m, 2H), 2.20(m, 1H), 2.05-1.15(m), 1.26(d, J=7Hz, 3H), 1.21(s, 3H), 1.20(s, 3H), 0.88(t, J=7Hz, 3H), 085(d, J=7Hz, 3H).

Step 7: Preparation of 6(R)-[2-[8(S)-(2,2-dimethylbutyryloxy)-2(S)-methyl-5-oxo-6(S)-methyl-1,2,3,4,4a(R)-,6,7,8,8a(R)-nonahydronaphthyl-1(S)]ethyl]-4(R)-hydroxy-3,4,5,6-tetrahydro-2H-pyran-2-one (13)

A stirred solution of compound 12 (150 mg, 0.34 mmol), 1,8-diazabicyclo[5,4,0]undec-7-ene (DBU) (52 µL, 0.34 mmol), and dry toluene was heated at 80°C for 3 hours. The cooled reaction mixture was concentrated and the residue subjected to flash chromatography (silica, 50% ethyl acetate/hexane) to give the desired compound 13 as a crystalline solid, (m.p. 133-134°C).
$^1$H NMR ($CDCl_3$) δ 5.28(m, 1H), 4.60(m, 1H), 4.48(m, 1H), 2.74(dd, J=18 and 5Hz, 1H), 2.62(m, 2H), 2.47-(ddd, J=9, 9, and 3Hz, 1H), 2.33(m, 1H), 2.00-1.10(m), 1.23(s, 3H), 1.22(s, 3H), 0.98(d, J=7Hz, 3H), 0.87(t, J=7Hz, 3H), 0.80(d, J=7Hz, 3H).

Step 8: Preparation of 6(R)-[2-[8(S)-(2,2-dimethylbutyryloxy)-2(S)-methyl-5(R)-hydroxy-6(S)-methyl-1,2,3,4,4a(R),6,7,8,8a(R)-decahydronaphthyl-1(S)]ethyl]-4(R)-hydroxy-3,4,5,6-tetrahydro-2H-pyran-2-one (14)

and

6(R)-[2-[8(S)-(2,2-dimethylbutyryloxy)-2(S)-methyl-5(S)-hydroxy-6(S)-methyl-1,2,3,4,4a(R),6,7,8,8a(R)-decahydronaphthyl-1(S)]ethyl]-4(R)-hydroxy-3,4,5,6-tetrahydro-2H-pyran-2-one (15)

To a stirred solution of compound 13 (68 mg, 0.15 mmol), THF (1.4 mL), and $H_2O$ (0.15 mL) at 0°C was added $NaBH_4$ (11 mg, 0.30 mmol). After 15 minutes, the reaction mixture was diluted with ethyl acetate, washed with $H_2O$ and brine, dried ($MgSO_4$) and concentrated. Flash chromatography (silica, 15% acetone/benzene) gave a faster moving compound 14 and a slower moving compound 15 as amorphous solids.
$^1$H NMR of compound (14) ($CDCl_3$) δ 5.08(m, 1H), 4.57(m, 1H), 4.36(m, 1H), 2.86(ddd, J=10, 5, and 5Hz, 1H), 2.74(dd, J=18 and 5Hz, 1H), 2.61(m, 1H), 2.05-1.15(m), 1.17(s, 6H), 1.00(d, J=7Hz, 3H), 0.85(t, J=7Hz, 3H), 0.83(d, J=7Hz, 3H).

| Elemental Anal. $C_{25}H_{42}O_6$ | | |
|---|---|---|
| Calc'd: | C, 68.46; | H, 9.65 |
| Found: | C, 68.17; | H, 9.50 |

$^1H$ NMR ($CDCl_3$) of compound (15) δ 5.14(m, 1H), 4.59(m, 1H), 4.36(m, 1H), 3.53(bs, 1H), 2.74(dd, J=18 and 5Hz, 1H), 2.60(m, 1H), 2.20(d, J=3Hz, 1H), 2.00-1.20(m), 1.17(s, 3H), 1.16(s, 3H), 0.95(d, J=7Hz, 3H), 0.85(t, J=7Hz, 3H), 0.84(d, J=7Hz, 3H).

| Elemental Anal. $C_{25}H_{42}O_6$ | | |
|---|---|---|
| Calc'd: | C, 68.46; | H, 9.65 |
| Found: | C, 68.09; | H, 9.18 |

EXAMPLE 4

Preparation of 6(R)-[2-[8(S)-2,2-dimethylbutyryloxy)-2(S)-methyl-5(R)-(1-phenylethyl-2-oxy)-6(R)-methyl-1,2,3,4,4a(R),5,6,7,8,8a(R)-decahydronaphthyl-1(S)]ethyl]-4(R)-hydroxy-3,4,5,6-tetrahydro-2H-pyran-2-one (18)

Example 1, Steps 1-6 were repeated but substituting tert-butyldiphenylsilyl as the hydroxy protecting group.

Step 7: 6(R)-[2-[8(S)-2,2-dimethylbutyryloxy)-2(S)-methyl-5(R)-(1-phenylethylen-2-oxy)-6(R)-methyl-1,2,3,4,4a(R),5,6,7,8,8a(R)-decahydronaphthyl-1(S)]ethyl]-4(R)-tert-butyldiphenylsilyloxy-3,4,5,6-tetrahydro-2H-pyran-2-one (16)

To a stirred solution of 6(R)-[2-[8(S)-(2,2-dimethylbutyryloxy)-2(S)-methyl-5(R)-hydroxy-6(R)-methyl-1,2,3,4,4a(R),5,6,7,8,8a(R)-decahydronaphthyl1(S)]ethyl]-4(R)-tert-butyldiphenylsilyloxy-3,4,5,6-tetrahydro-2H-pyran-2-one (270 mg, 0.40 mmol), β-methoxystyrene (165 µL, 1.2 mmol) and dry $CH_2Cl_2$ (4 mL) at 0°C was added (±)-camphorsulfonic acid (23 mg, 0.10 mmol). After 15 minutes, the cooling bath was removed and stirring continued for 3 hours. The reaction was quenched with $NEt_3$ (195 µL, 1.2 mmol) concentrated, and the residue subjected to flash chromatography (silica, 15% EtOAc/hexane) to afford compound 16 as a colorless foam.
$^1H$ NMR ($CDCl_3$) δ 7.68-7.20(m, 15H), 6.23(d, J=7Hz, 1H), 5.20)d, J=7Hz, 1H), 5.09(m, 1H), 4.67(m, 1H), 4.27(m, 1H), 3.56(dd, J=10 and 5Hz, 1H), 2.57(m, 1H), 2.43(dd, J=18 and 4Hz, 1H), 2.26(m, 1H), 2.10-1.10(m), 1.17(s, 3H), 1.16(s, 3H), 1.08(s, 9H), 0.86(t, J=7Hz, 3H), 0.84(d, J=7Hz, 3H).

Step 8: Preparation of 6(R)-[2-[8(S)-(2,2-dimethylbutyryloxy)-2(S)-methyl-5(R)-(1-phenylethyl-2-oxy)-6(R)-methyl-1,2,3,4,4a(R),5,6,7,8,8a(R)-decahydronaphthyl-1(S)]ethyl]-4(R)-tetrabutyldiphenylsilyloxy-3,4,5,6-tetrahydro-2H-pyran-2-one (17).

A mixture of compound 16 (150 mg, 0.19 mmol) 10% Pd/C (30 mg), and ethyl acetate (5.0 ml) was stirred at 25°C under a hydrogen atmosphere (1 atm) for 8.0 hours. The reaction mixture was filtered through a celite pad and concentrated. Flash chromatography (silica, 15% ethyl acetate/hexane) gave compound 17 as a colorless oil.
$^1H$ NMR ($CDCl_3$) δ 7.65-7.20(m, 15H), 5.00(m, 1H), 4.66 (m, 1H), 4.23(m, 1H), 3.78(m, 1H), 3.46(m, 1H), 3.02(dd, J=10 and 5Hz, 1H), 2.88(ddd, J=7,7, and 3Hz, 2H), 2.56(m, 1H), 2.41(dd, J=18 and 4Hz, 1H), 2.22(m, 1H), 2.05-1.10(m), 1.14(s, 3H), 1.08(s,9H), 0.98(d, J=7Hz, 3H), 0.82(t, J=7Hz, 3H), 0.79(d, J=7Hz, 3H).

Step 9: Preparation of 6(R)-[2-[8(S)-(2,2-dimethylbutyryloxy)-2(S)-methyl-5(R)-(1-phenylethyl-2-oxy)-6(R)-methyl-1,2,3,4,4a(R),5,6,7,8,8a(R)-decahydronaphthyl-1(S)]ethyl]-4(R)-hydroxy-3,4,5,6-tetrahydro-2H-pyran-2-one (18)

Utilizing the procedure of Example 1, Step 7 the compound 17 (39 mg, 50 mmol) was converted to the desired compound 18 which was a colorless oil.
$^1$H NMR ($CDCl_3$) $\delta$ 7.25(m, 5H), 5.05(m, 1H), 4.55(m, 1H), 4.32(m, 1H), 3.73(m, 1H), 3.47 (m, 1H), 3.01(dd, J=10 and 5Hz, 1H), 2.88 (m, 2H), 2.71(dd, J=18 and 5Hz, 1H), 2.59 (dd, J=18 and 4Hz, 1H), 2.22(m, 2H), 2.00-1.10(m), 1.14(s, 3H), 1.13(s, 3H), 0.99 (d, J=7Hz, 3H), 0.82(t, J=7Hz, 3H), 0.78(d, J=7Hz,3H).

| Elemental Analysis: $C_{33}H_{50}O_6 \cdot 0.25\ H_2O$ | | |
|---|---|---|
| Calc'd: | C, 72.43; | H, 9.32 |
| Found: | C, 72.53; | H, 9.32 |

EXAMPLE 5

Preparation of 6(R)-[2-[8(S)-(2,2-dimethylbutyryloxyl)-2(S)-methyl-5(R)-dibenzylaminocarbonyloxy-6(R)-methyl-1,2,3,4,4a(R),5,6,7,8,8a(R)-decahydronaphthyl-1(S)]ethyl]-4(R)-hydroxy-3,4,5,6-tetrahydro-2H-pyran-2-one (20)

Example 1, Steps 1-6 were repeated but substituting tert-butyldiphenylsilyl as the hydroxy protecting group.

Step 7: Preparation of 6(R)-[2-[8(S)-(2,2-dimethylbutyryloxy)-2(S)-methyl-5(R)-dibenzylaminocarbonyloxy-6-(R)-methyl-1,2,3,4,4a(R),5,6,7,8,8a(R)-decahydronaphthyl-1(S)]ethyl]-4(R) tert-butyldiphenylsilyloxy-3,4,5,6-tetrahydro-2H-pyran-2-one (19).

A solution of 6(R)-[2-[8(S)-(2,2-dimethylbutryloxy)-2(S)-methyl-5(R)-hydroxy-6(R)-methyl-1,2,3,4,4a(R)-,5,6,7,8,8a(R)-decahydronaphthyl-1(S)]ethyl]-4(R)-tert-butyldiphenylsilyloxy-3,4,5,6-tetrahydro-2H-pyran-2-one (25 mg, 37 mmol), triethylamine (21 $\mu$L, 0.15 mmol), and dry $CH_2Cl_2$ (200 $\mu$L) was added dropwise to a stirred solution of phosgene (20% in toluene, 67 $\mu$L, 0.15 mmol) and $CH_2Cl_2$ (600 $\mu$L) at 0°C. After 5 minutes the cooling bath was removed and the reaction mixture stirred for 20 minutes. Concentration in situ followed by sequential addition of $CH_2Cl_2$ (400 $\mu$L) and dibenzylamine (8 $\mu$L, 41 mmol) at ambient temperature, resulted in a heterogeneous mixture. After 15 minutes the reaction mixture was diluted with ether, washed with $H_2O$ and brine, dried ($MgSO_4$), and concentrated. Flash chromatography (silica, 15-20% ethyl acetate/hexane) gave compound 19 as an oil.
$^1$H NMR ($CDCl_3$) $\delta$ 7.63-7.26(m, 20H), 5.12(m, 1H), 4.75(dd, J=10 and 5Hz, 1H), 4.60(m, 1H), 4.40 (m, 2H), 4.33(m, 1H), 2.60(m, 2H), 2.20-1.10(m), 1.17(s, 3H), 1.16(s, 3H), 1.07(s, 9H), 1.00 (d, J=7Hz, 3H), 0.80(m, 6H).

Step 8: Preparation of 6(R)-[2-[8(S)-(2,2-dimethylbutyryloxyl)-2(S)-methyl-5(R)-dibenzylaminocarbonyloxy-6-(R)-methyl-1,2,3,4,4a(R),5,6,7,8,8a(R)-decahydronaphthyl-1(S)]ethyl]-4(R)-hydroxy-3,4,5,6-tetrahydro-2H-pyran-2-one (20).

Compound 19 (72 mg, 79 mmol) was dissolved in a premixed solution of tetrabutylammonium flouride (1M in THF, 300 $\mu$L, 0.3 mmol), HOAc (20 mL, 0.3 mmol), and THF (300 $\mu$L) followed by heating at 50°C for 1.0 hour. The cooled reaction mixture was diluted with ether, washed with $H_2O$ and brine, dried ($MgSO_4$), and concentrated. Flash chromatography (silica, 60% EtOAc/hexane) gave compound 20 as a colorless foam.
$^1$H NMR ($CDCl_3$) $\delta$ 7.37-7.18(m, 10H), 5.11(m, 1H), 4.75(dd, J=10 and 5Hz, 1H), 4.59(m, 1H), 4.47 (m, 3H), 4.34(m, 1H), 2.72(dd, J=18 and 5Hz, 1H),2.61(dd, J=18 and 3Hz, 1H), 2.32(m, 1H), 2.00-1.10(m), 1.16(s, 3H), 1.15(s, 3H), 1.00(d, J=7Hz, 3H), 0.84(t, J=7Hz, 3H), 0.83(d, J=7Hz, 3H).

| Elemental Analysis: $C_{40}H_{55}O_7N \bullet 0.5\ H_2O$ | | | |
|---|---|---|---|
| Calc'd: | C, 71.61; | H, 8.41; | N, 2.09 |
| Found: | C, 71.66; | H, 8.31; | N, 2.04 |

EXAMPLE 6

Preparation of 6(R)-[2-[8(S)-(2,2-dimethylbutyryloxy)-2(S)-methyl-5(R)-diphenylphosphinyloxy-6(R)-methyl-1,2,3,4,4a(R),5,6,7,8,8a(R)-decahydronaphthyl-1(S)}ethyl]-4(R)-hydroxy-3,4,5,6-tetrahydro-2H-pyran-2-one (22)

Example 1, Steps 1-6 were repeated but substituting tert-butyldiphenylsilyl as the hydroxyl protecting group.

Step 7: Preparation of 6(R)-[2-[8(S)-(2,2-dimethylbutyryloxy)-2(S)-methyl-5(R)-diphenylphosphinyloxy-6(R)-methyl-1,2,3,4,4a(R),5,6,7,8,8a(R)-decahydronaphthyl-1(S)]ethyl]-4(R)-tert-butyldiphenylsilyloxy-3,4,5,6-tetrahydro-2H-pyran-2-one (21).

To a stirred solution of 6(R)-[2-[8(S)-(2,2-dimethylbutyryloxy)-2(S)-methyl-5(R)-hydroxy-6(R)-methyl-1,2,3,4,4a(R),5,6,7,8,8a(R)-decahydronaphthyl-1(S)]ethyl]-4(R)-tert-butyldiphenylsilyloxy-3,4,5,6-tetrahydro-2H-pyran-2-one (59 mg, 87 µmol) N,N-dimethyl aminopyridine (DMAP) (43 mg, 0.35 mmol), and $CH_2Cl_2$ - (0.44 mL) at ambient temperature was added diphenyl phosphosphinic chloride (33 µL, 0.17 mmol). After 20 minutes the reaction mixture was diluted with ether, washed with $H_2O$ and brine, dried ($MgSO_4$), and concentrated. Flash chromatography (silica, 45% EtOAc/hexane) gave compound 21 as an oil.
$^1$H NMR ($CDCl_3$) δ 7.85-7.25(m, 20H), 4.98(m, 1H), 4.64(m, 1H), 4.28(m, 2H), 2.55(m, 1H), 2.39(dd, J=18 and 4Hz, 1H), 2.05-1.10(m), 1.14(s, 3H), 1.13(s, 3H), 1.12(d, J=7Hz, 3H), 1.03(s, 9H), 0.81(t, J=7Hz, 3H), 0.73(d, J=7HZ, 3H).

Step 8: Preparation of 6(R)-[2-[8(S)-(2,2-dimethylbutyryloxy)-2(S)-methyl-5(R)-diphenylphosphinyloxy-6(R)-methyl-1,2,3,4,4a(R),5,6,7,8,8a(R)-decahydronaphthyl-1(S)]ethyl]-4(R)-hydroxy-3,4,5,6-tetrahydro-2H-pyran-2-one (22)

To a stirred solution of compound 21 (64 mg 73 µmol), THF (0.3 mL), and HOAc (17 µL, 0.3 mmol) was added tetrabutylammonium fluoride (1M in THF, 300 µL, 0.3 mmol) followed by heating at 50 °C. After 3.0 hours the cooled reaction mixture was diluted with ether, washed with $H_2O$ and brine, dried ($MgSO_4$), and concentrated. Flash chromatography (silica, 80% ethyl acetate/hexane) gave compound 22 as a colorless oil.
$^1$H NMR ($CDCl_3$) δ 7.80(m, 4H), 7.46(m, 6H), 5.01(m,1H), 4.54(m, 1H), 4.30(m, 1H), 4.27(m, 1H), 2.62(m, 3H), 2.10-1.10(m), 1.14(s, 3H), 1.13(s, 3H), 1.12 (d, J=7Hz, 3H), 0.82(t, J=7Hz, 3H), 0.73(d, J=7Hz, 3H).

| Elemental Analysis: $C_{37}H_{51}O_7P \bullet 0.5\ H_2O$ | | |
|---|---|---|
| Calc'd: | C, 68.60; | H, 8.09 |
| Found: | c, 68.69; | H, 8.03 |

EXAMPLE 7

6(R)-[2-[8(S)-(2,2-dimethylbutyryloxy)-2(S)-methyl-5(R)-hydroxy-6(R)-methyl-1,2,4a(R),5,6,7,8,8a(R)-octahydronaphthyl-1(S)]ethyl]-4(R)-hydroxy-3,4,5,6-tetrahydro-2H-pyran-2-one; and the corresponding ring opened dihydroxy acids and esters thereof.

Step 1: Preparation of 6(R)-[2-[8(S)-(2,2-dimethylbutyryloxy)-2(S)-methyl-6(R)-methyl-1,2,6,7,8,8a(R)-hexahydronaphthyl-1(S)]ethyl]-4(R)-tert-butyldiphenylsilyloxy-3,4,5,6-tetrahydro-2H-pyran-2-one (23).

To a stirred solution of Simvastatin (20.0 g, 48 mmol), imidazole (8.2 g, 0.12 mol), and dry DMF (100 mL) at 25°C was added tert-butyldiphenylsilylchloride (13.0 mL, 50 mmol). After stirring at 25°C for 18 hours the reaction mixture was diluted with pet, ether, washed with $H_2O$ (2X) and brine, dried ($MgSO_4$), and concentrated to furnish (23) as a colorless oil.
TLC $R_f$ = 0.75 (silica, 30% ethyl acetate/hexanes);
$^1H$ NMR ($CDCl_3$) δ 7.63 (m, 4H), 7.42 (m, 6H), 6.00 (d, J = 10 Hz, 1H), 5.80 (dd, J = 10 and 6 Hz, 1H), 5.54 (m, 1H), 5.34 (m, 1H), 4.71 (m, 1H), 4.28 (m, 1H), 2.63-2.23 (m), 2.08-1.20 (m), 1.15 (s, 3H), 1.14 (s, 3H), 1.14 (d, J = 7Hz, 3H), 1.09 (s, 9H), 0.91 (d, J = 7Hz, 3H), 0.84 (t, J = 7Hz, 3H).

Step 2: Preparation of 6(R)-[2-[8(S)-(2,2-dimethylbutyryloxy)-2(S)-methyl-4a(R),5(S)-epoxy-6(R)-methyl-1,2,6,7,8,8a(R)-hexahydronaphthyl-1(S)]ethyl]-4(R)-tert-butyldiphenylsilyloxy-3,4,5,6-tetrahydro-2H-pyran-2-one (24).

To a stirred mixture of (23) (47.0 g, 72 mmol), $NaHCO_3$ (12.0 g, 0.14 mol), and EtOAc (600 mL) at 0°C was added 55% meta-chloroperbenzoic acid (27.0 g, 86 mmol). After 1.0 h at 0°C the reaction mixture was diluted with EtOAc, washed sequentially with 10% $Na_2SO_3$, $H_2O$, and brine, dried ($MgSO_4$), and concentrated. Flash chromatography (silica, 10% ethyl acetate/hexanes w/2% triethylamine) gave (24) as an oil.
TLC $R_f$ = 0.51 (silica, 30% ethyl acetate/hexanes);
$^1H$ NMR ($CDCl_3$) δ 7.60 (m, 4H), 7.40 (m, 6H), 6.21 (dd, J = 10 and 6Hz, 1H), 5.12 (d, J = 10 Hz, 1H), 5.11 (m, 1H), 4.68 (m, 1H), 4.24 (m, 1H), 2.96 (s, 1H), 2.60-2.28 (m, 5H), 2.01 (dd, J = 12 and 4Hz, 1H), 1.90-1.20 (m), 1.16 (d, J = 7Hz, 3H), 1.12 (s, 3H), 1.10 (s, 3H) 1.04 (s, 9H), 0.96 (d, J = 7Hz, 3H), 0.80 (t, J = 7Hz, 3H).

Step 3: Preparation of 6(R)-[2-[8(S)-(2,2-dimethylbutyryloxy)-2(S)-methyl-5-oxo-6(R)-methyl-1,2,4a(R)-,6,7,8,8a(R)-heptahydronaphthyl-1(S)]ethyl]-4(R)-tert-butyldiphenylsilyloxy-3,4,5,6-tetrahydro-2H-pyran-2-one (25).

A stirred solution of (24) (20.0 g, 30.0 mmol), toluene (150 mL), and ether (150 mL) at -15°C was treated dropwise with boron trifluoride etherate (3.0 mL, 24.4 mmol) over a 5 minute period. After stirring for 20 minutes the reaction mixture was diluted with ether, washed with sat. $NaHCO_3$, $H_2O$, and brine, dried ($MgSO_4$), and concentrated. Flash chromatography (silica, 12% ethylacetate/hexanes) furnished crude (25) as an oil.
TLC $R_f$ = 0.42 (silica, 30% ethyl acetate/hexanes);
$^1H$ NMR ($CDCl_3$) δ 7.62 (m, 4H) 7.40 (m, 6H), 5.98 (d, J = 10 Hz, 1H), 5.84 (m, 1H), 5.31 (m, 1H), 4.72 (m, 1H), 4.18 (m, 1H), 3.48 (bd, J = 10 Hz, 1H), 2.63-2.23 (m, 3H), 1.95-1.10 (m), 1.25 (d, J = 7Hz, 3H), 1.19 (s, 3H), 1.18 (s, 3H), 1.10 (s, 9H), 0.88 (t, J = 7Hz, 3H), 0.85 (d, J = 7Hz, 3H).

Step 4: Preparation of 6(R)-[2-[8(S)-(2,2-dimethylbutyryloxy)-2(S)-methyl-5(R)-hydroxy-6(R)-methyl-1,2,4a-(R),5,6,7,8,8a(R)-octahydronaphtyl-1(S)]ethyl]-4(R)-tert-butyldiphenylsilyloxy-3,4,5,6-tetrahydro-2H-pyran-2-one (26).

To a stirred solution of (25) (6.4 g, 9.5 mmol), THF (90 mL), and $H_2O$ (5 mL) at 0°C was added 0.36 g (0.95 mmol) $NaBH_4$ in one portion. After 20 minutes at 0°C the reaction mixture was diluted with ether, washed with $H_2O$ (2X) and brine, dried ($MgSO_4$), and concentrated. Flash chromatography (silica, 20% ethyl acetate/hexanes) gave (26) as a colorless oil.
TLC $R_f$ = 0.30 (silica, 35% ethyl acetate/hexanes);
$^1H$ NMR ($CDCl_3$) δ 7.62 (m, 4H), 7.45 (m, 6H), 5.96 (d, J = 10 Hz, 1H), 5.80 (m, 1H), 5.08 (m, 1H), 4.72 (m, 1H), 4.28 (m, 1H), 3.47 (m, 1H), 2.62-2.10 (m, 5H), 1.85-1.20 (m), 1.16 (s, 3H), 1.15 (s, 3H), 1.10 (d, J = 7Hz, 3H), 1.07 (s, 9H), 0.85 (t, J = 7Hz, 3H), 0.85 (t, J = 7Hz, 3H).

Step 5: Preparation of 6(R)-[2-[8(S)-(2,2-dimethylbutyryloxy)-2(S)-methyl-5(R)-hydroxy-6(R)-methyl-1,2,4a-(R),5,6,7,8,8a(R)-octahydronaphthyl-1(S)]ethyl]-4(R)-hydroxy-3,4,5,6-tetrahydro-2H-pyran-2-one (I-27).

A premixed solution of tetrabutylammonium fluoride (1M in THF, 16 mL, 16.0 mmol) and HOAc (0.92 mL, 16.0 mmol) was added in one portion to a stirred solution of (26) (3.6 g, 5.3 mmol) in THF (32 mL)

followed by heating at 50°C for 3.0 hours. The cooled reaction mixture was diluted with ether, washed with $H_2O$ (2X) and brine, dried ($MSO_4$), and concentrated. Flash chromatography (silica, 70% ethyl acetate/hexanes) gave (I-27) as a solid. Recrystallization (ethyl acetate/hexanes) gave (I-27) as colorless needles mp = 130-132°C.
TLC $R_f$ = 0.39 (silica, ethyl acetate);
$^1H$ NMR ($CDCl_3$) δ 5.95 (d, J = 12 Hz, 1H), 5.79 (m, 1H), 5.11 (m, 1H), 4.60 (m, 1H), 4.38 (m, 1H), 3.49 (dt, J = 11 and 6 Hz, 1H), 2.74 (dd, J = 17 and 5Hz, 1H), 2.61 (m, 1H), 2.48 (m, 1H), 2.30 (m, 1H), 2.18 (m, 1H), 2.05-1.20 (m), 1.17 (s, 3H), 1.16 (s, 3H), 1.09 (d, J = 7Hz, 3H), 0.86 (t, J = 7Hz, 3H), 0.85 (d, J = 7Hz, 3H).

## EXAMPLE 8

Preparation of 6(R)-[2-[8(S)-(2,2-dimethylbutyryloxy)-2(S)-methyl-5-oxo-6(R)-methyl-1,2,3,6,7,8,8a(R)-heptahydronaphthyl-1(S)]ethyl]-4(R)-tert-butyldimethylsilyloxy-3,4,5,6-tetrahydro-2H-pyran-2-one (11)

A mixture of the bromoketone 4, (50 mg, 79 µmol), from Example 1, step 4, 1,2-dichloroethane (1.0 mL), and 2,6-lutidine (17 µL, 150 mmol) at 25°C was treated with $AgNO_3$(25 mg, 150 mmol). After 2.0 hours the heterogeneous mixture was diluted with ether, washed with $H_2O$ and brine, dried ($MgSO_4$), and concentrated. Flash chromatography (silica, 15% EtOAc/hexanes) gave compound 11 as an oil.
'H NMR ($CDCl_3$) δ 6.77 (m,1H), 5.40(m,1H), 4.62 (m,1H), 4.31(m,1H) 2.75-1.10(m), 1.15 (d,J=7Hz, 3H), 1.13-(s,3H), 0.91(s,9H), 0.81(t,J=7Hz, 3H), 0.79 (d,J=Hz,3H), 0.10(s,6H).

## EXAMPLES 9-40

Utilizing the general procedures of Examples 1-8, the following compounds of formula (I) are prepared from the appropriately substituted starting materials.

| Compound No. | $R_4$ | $R_5$ | $R_6$ | a |
|---|---|---|---|---|
| 28 | $CH_3$ | H | $O_2COCH_3$ | - |
| 29 | $CH_3$ | $O_2COCH_3$ | H | - |

| Compound No. | $R_4$ | $R_5$ | $R_6$ | a |
|---|---|---|---|---|
| 30 | $CH_3$ | H | $O_2CCH_3$ | - |
| 31 | $CH_3$ | $O_2CCH_3$ | H | - |
| 32 | H | OH | H | - |
| 33 | H | H | OH | - |
| 34 | $CH_2OH$ | OH | H | - |
| 35 | $CH_2OH$ | H | OH | - |
| 36 | $CH_2Ph$ | OH | H | - |
| 37 | $CH_2Ph$ | H | OH | - |
| 38 | $CH_2O_2CPh$ | H | OH | - |
| 39 | $CH_2O_2CPh$ | OH | H | - |
| 40 | $CH_2OH$ | O | O | - |
| 41 | $CH_2Ph$ | O | O | - |
| 42 | H | O | O | - |
| 43 | $CH_2OH$ | $OCH_2Ph$ | H | - |
| 44 | $CH_2OH$ | H | $OCH_2Ph$ | - |
| 45 | $CH_3$ | $OCH_2CH_3$ | H | - |
| 46 | $CH_3$ | H | $OCH_2CH_3$ | - |
| 47 | $(CH_2)_2$ | OH | H | - |
| 48 | $(CH_2)_2$ | H | OH | - |
| 49 | $CH_3$ | $(CH_2)_2$ | | - |
| 50 | $CH_2OH$ | OH | H | db |
| 51 | $CH_2OH$ | H | OH | db |
| 52 | H | OH | H | db |
| 53 | H | H | OH | db |
| 54 | $CH_2Ph$ | OH | H | db |
| 55 | $CH_2Ph$ | H | OH | db |
| 56 | $CH_3$ | $OCH_2CH_3$ | H | db |
| 57 | $CH_3$ | H | $OCH_2CH_3$ | db |
| 58 | $CH_3$ | H | $O_2COCH_3$ | db |

- = single bond
db = double bond
$R_5 = R_6 = O$ means C=O

EXAMPLE 41

Preparation of Ammonium Salts of Compounds II

The lactone (1.0 mmol) from Example 1 Step 7 is dissolved with stirring in 0.1N NaOH (1.1 mmol) at ambient temperature. The resulting solution is cooled and acidified by the dropwise addition of 1N HCl. The resulting mixture is extracted with diethyl ether and the extract washed with brine and dried ($MgSO_4$). The $MgSO_4$ is removed by filtration and the filtrate saturated with ammonia (gas) to give the ammonium salt.

EXAMPLE 42

Preparation of Alkali and Alkaline Earth Salts of Compounds II

To a solution of 44 mg of lactone from Example 1 Step 7 in 2 ml of ethanol is added 1 ml of aqueous 0.1N NaOH. After one hour at room temperature, the mixture is taken to dryness in vacuo to yield the desired sodium salt.

In like manner, the potassium salt is prepared using one equivalent of potassium hydroxide, and the calcium salt, using one equivalent of CaO.

EXAMPLE 43

Preparation of Ethylenediamine Salts of Compounds II

To solution of 0.50 g of the ammonium salt from Example 41 in 10 ml of methanol is added 0.04 ml of ethylenediamine. The methanol is stripped off under vacuum to obtain the desired ethylenediamine salt.

EXAMPLE 44

Preparation of Tris(hydroxymethyl)aminomethane Salts of Compounds II

To a solution of 202 mg of the ammonium salt from Example 41 in 5 ml of methanol is added a solution of 50 mg of tris(hydroxymethyl)aminomethane is 5 ml of methanol. The solvent is removed in vacuo to afford the desired tris(hydroxymethyl)aminomethane salt.

EXAMPLE 45

Preparation of L-Lysine Salts of Compounds II

A solution of 0.001 mole of L-lysine and 0.0011 mole of the ammonium salt from Example 41 in 15 ml of 85% ethanol is concentrated to dryness in vacuo to give the desired L-lysine salt.

Similarly prepared are the L-arginine, L-ornithine, and N-methyglucamine salts.

EXAMPLE 46

Preparation of Tetramethylammonium Salts of Compounds II

A mixture of 68 mg of ammonium salt from Example 41 in 2 ml of methylene chloride and 0.08 ml of 24% tetramethylammonium hydroxide in methanol is diluted with ether to yield the desired tetramethylammonium salt.

EXAMPLE 47

Preparation of Methyl Esters of Compounds II

To a solution of 400 mg of lactone from Example 1 Step 7 in 100 ml of absolute methanol is added 10 ml 0.1 M sodium methoxide in absolute methanol. This solution is allowed to stand at room remperature for one hour, then is diluted with water and extracted twice with ethyl acetate. The organic phase is separated, dried ($Na_2SO_4$), filtered and evaporated in vacuo to yield the desired methyl ester.

In like manner, by the use of equivalent amounts of the alkoxides derived from propanol, butanol, isobutanol, t-butanol, amyl alcohol, isoamyl alcohol, 2-dimethylaminoethanol, benzyl alcohol, phenethanolm 2-acetamidoethanol and the like, and employing the corresponding alcohol, phenethanol, 2-acetamidoethanol and the like, and employing the corresponding alcohol as solvent, the corresponding esters are obtained.

EXAMPLE 48

Preparation of Free Dihydroxy Acids

The sodium salt of the compound II from Example 42 is dissolved in 2 ml of ethanol-water (1:1, v:v) and added to 10 ml of 1N hydrochloric acid from which the dihydroxy acid is extracted with ethyl acetate. The organic extract is washed once with water, dried ($Na_2SO_4$), and evaporated in vacuo with a bath temperature not exceeding 30 ° C. The dihydroxy acid derivative derived slowly reverts to the corresponding parent lactone on standing at room temperature. The dihydroxy acid form can be maintained by increasing the pH above 7.0.

EXAMPLE 49

As a specific embodiment of a composition of this invention, 20 mg of lactone from Example 1 Step 7 is formulated with sufficient finely divided lactose to provide a total amount of 580 to 590 mg to fill a size 0, hard-gelatin capsule.

## Claims

**1.** A Compound represented by the structural formulae (I) or (II):

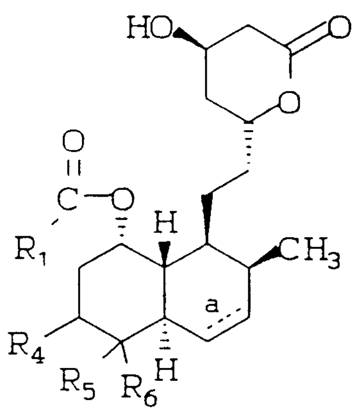

(I)

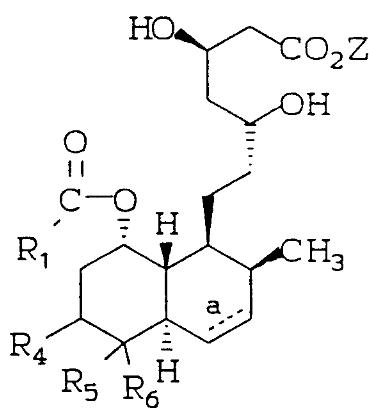

(II)

wherein:

$R_1$ is selected from:

(1) $C_{1-10}$ alkyl;

(2) substituted $C_{1-10}$ alkyl in which one or more substituent(s) is selected from

(a) halogen,
(b) hydroxy,
(c) $C_{1-10}$ alkoxy,
(d) $C_{1-5}$ alkoxycarbonyl,
(e) $C_{1-5}$ acyloxy,
(f) $C_{3-8}$ cycloalkyl,

(g) phenyl,
(h) substituted phenyl in which the substituents are X and Y,
(i) $C_{1-10}$ alkylS(0)$_n$ in which n is 0 to 2,
(j) $C_{3-8}$ cycloalkylS(0)$_n$,
(k) phenylS(0)$_n$,
(l) substituted phenylS(0)$_n$ in which the substituents are X and Y, and
(m) oxo;
(3) $C_{1-10}$ alkoxy;
(4) $C_{2-10}$ alkenyl;
(5) $C_{3-8}$ cycloalkyl;
(6) substituted $C_{3-8}$ cycloalkyl in which one substituent is selected from
(a) $C_{1-10}$ alkyl
(b) substituted $C_{1-10}$ alkyl in which the substituent is selected from
(i) halogen,
(ii) hydroxy,
(iii) $C_{1-10}$ alkoxy,
(iv) $C_{1-5}$ alkoxycarbonyl,
(v) $C_{1-5}$ acyloxy,
(vi) phenyl,
(vii) substituted phenyl in which the substituents are X and Y
(viii) $C_{1-10}$ alkylS(O)$_n$,
(ix) $C_{3-8}$ cycloalkylS(O)$_n$,
(x) phenylS(O)$_n$,
(xi) substituted phenylS(O)$_n$ in which the substituents are X and Y, and
(xii) oxo,
(c) $C_{1-10}$ alkylS(0)$_n$,
(d) $C_{3-8}$ cycloalkylS(0)$_n$,
(e) phenylS(0)$_n$,
(f) substituted phenylS(0)$_n$ in which the substituents are X and Y,
(g) halogen,
(h) hydroxy,
(i) $C_{1-10}$ alkoxy,
(j) $C_{1-5}$ alkoxycarbonyl,
(k) $C_{1-5}$ acyloxy,
(l) phenyl, and
(m) substituted phenyl in which the substituents are X and Y:
(7) phenyl;
(8) substituted phenyl in which the substituents are X and Y;
(9) amino;
(10) $C_{1-5}$ alkylamino;
(11) di($C_{1-5}$ alkyl)amino;
(12) phenylamino;
(13) substituted phenylamino in which the substituents are X and Y;
(14) phenyl $C_{1-10}$ alkylamino;
(15) substituted phenyl $C_{1-10}$ alkylamino in which the substituents are X and Y;
(16) a member selected from
(a) piperidinyl,
(b) pyrrolidinyl,
(c) piperazinyl,
(d) morpholinyl, and
(e) thiomorpholinyl; and
(17) $R_{10}S$ in which $R_{10}$ is selected from
(a) $C_{1-10}$ alkyl,
(b) phenyl, and
(c) substituted phenyl in which the substituents are X and Y;

$R_4$ is;

(1) halogen;
(2) $C_{1-10}$ alkyl; and
(3) substituted $C_{1-10}$ alkyl in which one or more substituents is selected from
(a) hydrogen,
(b) hydroxy,
(c) $C_{1-10}$ alkoxy
(d) $C_{1-5}$ alkoxycarbonyl,
(e) $C_{1-5}$ alkylacyloxy,
(f) phenylacyloxy,
(g) phenoxycarbonyl,
(h) phenyl $C_{1-5}$ alkylacyloxy,
(i) phenyl $C_{1-5}$ alkoxy,
(j) amino,
(k) $C_{1-5}$ alkylamino,
(l) di($C_{1-5}$ alkyl)amino,
(m) phenylamino,
(n) substituted phenylamino in which the substituents are X and Y;
(o) phenyl $C_{1-5}$ alkylamino,
(p) substituted phenyl $C_{1-5}$ alkylamino in which the substituents are X and Y,
(q) $C_{3-8}$ cycloalkyl,
(r) phenyl,
(s) substituted phenyl in which the substituents are X and Y,
(t) phenylS$(O)_n$,
(u) substituted phenyl S$(O)_n$ in which the substituents are X and Y,
(v) phenyl $C_{1-5}$ alkyl S$(O)_n$,
(w) $C_{1-5}$ alkylS$(O)_n$;
(x) phenylaminoacyloxy,
(y) $C_{1-5}$alkylaminoacyloxy,
(z) $C_{1-5}$alkylacylamino,
(aa) di(phenyl$C_{1-5}$alkyl)phosphonyl
(bb) di($C_{1-5}$alkyl)phosphinyl
(4) $R_4$ together with the carbon atom to which it is attached represents a $C_{3-8}$ carbocyclic ring;

$R_5$ and $R_6$ independently are H, OH, $OR_7$ or $R_5$ and $R_6$ together with the carbon to which they are attached represent C=O or $R_5$ and $R_6$ together with the carbon to which they are attached represent a carbocyclic ring of 3 to 7 atoms; provided that when $R_5$ is H, $R_6$ is OH or $OR_7$, and when $R_5$ is OH, $R_6$ is H, and when $R_5$ is $OR_7$, $R_6$ is H;

$R_7$ is

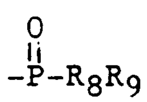, 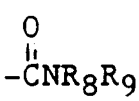 or 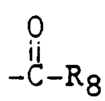, 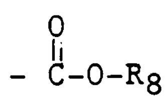,

phenyl$C_{1-3}$alkyl, $C_{1-5}$alkyl;

$R_8$ and $R_9$ independently are H, $C_{1-3}$alkyl, phenyl$C_{1-3}$alkyl or aryl wherein aryl is phenyl, naphthyl, pyridyl, furanyl, thienyl or phenyl, naphthyl, pyridyl, furanyl or thienyl substituted with groups X and Y; provided that when $R_7$ is

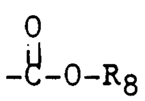,

$R_8$ is not H and when $R_7$ is

$$-\overset{\overset{\displaystyle O}{\|}}{P}-R_8R_9$$

neither $R_8$ nor $R_9$ is H;

X and Y are independently selected from:

a) OH;
b) halogen,
c) trifluoromethyl,
d) $C_{1-3}$alkoxy,
e) $C_{1-3}$alkylocarbonyloxy,
f) phenylcarbonyloxy,
g) $C_{1-3}$alkoxycarbonyl,
h) phenyloxycarbonyl,
i) hydrogen,
j) $C_{1-5}$alkyl;

Z is selected from:

(1) hydrogen;
(2) $C_{1-5}$alkyl;
(3) substituted $C_{1-5}$ in which the substituent is selected from:
   (a) phenyl,
   (b) dimethylamino, and
   (c) acetylamino, and
(4) 2,3 dihydroxypropyl;

halogen is Cl or F;

a is a single bond or a double bond; provided that when a is a double bond and $R_5$ and $R_6$ is OH, the configuration of the 5-position of the naphthyl ring is 5(R); or a pharmaceutically acceptable salt thereof.

**2.** A Compound of Claim 1 wherein:

$R_4$ is;

(1) hydrogen;
(2) $C_{1-10}$ alkyl;
(3) substituted $C_{1-10}$ alkyl in which one or more substituents is selected from:
   (a) halogen,
   (b) hydroxy,
   (c) amino;
(4) $CH_2R_{12}$ in which $R_{12}$ is selected from:
   (a) $C_{1-5}$ alkoxy,
   (b) $C_{1-5}$ alkoxy carbonyl,
   (c) $C_{1-5}$ alkylacyloxy,
   (d) phenylacyloxy,
   (e) phenoxycarbonyl,
   (f) phenyl$C_{1-5}$alkyl,
   (g) phenyl$C_{1-5}$alkoxy
   (h) $C_{1-5}$alkylamino,
   (i) di($C_{1-5}$alkyl)amino,
   (j) phenylamino,
   (k) substituted phenylamino in which the substituents are X and Y,
   (l) phenyl $C_{1-5}$alkylamino,
   (m) substituted phenyl$C_{1-5}$alkyl amino in which the substituents are X and Y,
   (n) $C_{3-8}$ cycloalkyl,
   (o) phenyl,
   (p) substituted phenyl in which the substituents are X and Y,
   (q) phenyl$S(O)_n$,
   (r) substituted phenyl$S(O)_n$ in which the substituents are X and Y,

(s) phenyl $C_{1-5}$ alkylS(O)$_n$
(t) $C_{1-5}$ alkylS(O)$_n$,
(u) phenylaminoacyloxy,
(v) $C_{1-5}$ alkylaminoacyloxy,
(w) $C_{1-5}$ alkylacylamino,
(x) di(phenyl$C_{1-5}$alkyl)phosphonyl,
(y) di($C_{1-5}$alkyl)phosphinyl;

(5) $R_4$ together with the carbon atom to which it is attached represents a cyclopropane ring;

$R_5$ and $R_6$ independently are H, OH, $OR_7$ or $R_5$ and $R_6$ together with the carbon to which they are attached represent C=O, or $R_5$ and $R_6$ together with the carbon to which they are attached represent a cyclopropane ring; provided that when $R_5$ is H, $R_6$ is OH or $OR_7$ and when $R_5$ is OH, $R_6$ is H,. and when $R_5$ is $OR_7$, $R_6$ is H;

X and Y are independently selected from:
a) OH,
b) F,
c) trifluoromethyl,
d) $C_{1-3}$alkoxy,
e) hydrogen,
f) $C_{1-5}$alkyl.

**3.** A Compound of Claim 2 wherein:
$R_1$ is $C_{1-10}$alkyl; and
$R_4$ is $CH_3$, H, or $CH_2$phenyl;
$R_7$ is

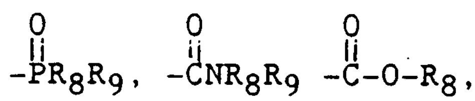

$C_{1-5}$alkyl or phenyl$C_{1-3}$ alkyl;
$R_8$ and $R_9$ independently are H, $C_{1-3}$alkyl, phenyl$C_{1-3}$alkyl or aryl wherein aryl is phenyl or naphthyl or phenyl or naphthyl substituted with X.

**4.** A Compound of Claim 3 wherein:
$R_1$ is 2-butyl or 2-methyl-2-butyl;
$R_4$ is $CH_3$.

**5.** A Compound of Claim 2 wherein:
$R_1$ is $C_{1-10}$alkyl;
$R_4$ is $CH_2OH$ or phenylacyloxymethyl;
$R_7$ is

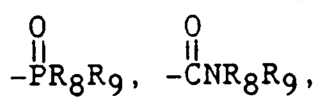

$C_{1-5}$alkyl or phenyl$C_{1-5}$alkyl;
$R_8$ and $R_9$ independently are H, $C_{1-3}$alkyl, phenyl$C_{1-3}$alkyl or aryl wherein aryl is phenyl or naphthyl or phenyl or naphthyl substituted with X.

**6.** A Compound of Claim 5 wherein:
$R_1$ is 2-butyl or 2-methyl-2-butyl;
$R_4$ is $CH_2OH$.

**7.** A Compound of Claim 4 selected from: 6(R)-[2-[8(S)-(2,2-dimethylbutyryloxy)-2(S)-methyl-5(R)-hydroxy-6(R)-methyl-1,2,3,4,4a(R),5,6,7,8,8a(R)-decahydronaphthyl-1(S)]ethyl]-4(R)-hydroxy-3,4,5,6-tetrahydro-2H-pyran-2-one;
6(R)-[2-[8(s)-(2,2-dimethylbutyryloxy)-2(S)-methyl-5(S)-hydroxy-6(S)-methyl-1,2,3,4,4a(R),5,6,7,8,8a(R)-decahydronaphthyl-1(S)]ethyl]-4(R)-hydroxy-3,4,5,6-tetrahydro-2H-pyran-2-one;
6(R)-[2-[8(S)-(2,2-dimethylbutyryloxy)-2(S)-methyl-5(R)-benzylaminocarbonyloxy-6(R)-methyl-1,2,3,4,4a-(R),5,6,7,8,8a(R)-decahydronaphthyl-1(S)]ethyl]-4(R)-hydroxy-3,4,5,6-tetrahydro-2H-pyran-2-one;
6(R)-[2-[8(S)-(2,2-dimethylbutyryloxy)-2(S)-methyl-5(R)-diphenylphosphinyloxy-6(R)-methyl-1,2,3,4,4a-(R),5,6,7,8,8a(R)-decahydronaphthyl-1(S)]ethyl]-4(R)-hydroxy-3,4,5,6-tetrahydro-2H-pyran-2-one;
6(R)-[2-[8(S)-(2,2-dimethylbutyryloxy)-2(S)-methyl-5(R)-(1-phenylethyl-2-oxy)-6(R)-methyl-1,2,3,4,4a(R)-,5,6,7,8,8a(R)-decahydronaphthyl-1(S)}ethyl}-4(R)-hydroxy-3,4,5,6-tetrahydro-2H-pyran-2-one;
6(R)-[2-[8(S)-(2,2-dimethylbutyryloxy)-2(S)-methyl-5(R)-dibenzylaminocarbonyloxy-6(R)-methyl-1,2,3,4,4a(R),5,6,7,8,8a(R)-decahydronaphthyl-1(S)]ethyl]-4(R)-hydroxy-3,4,5,6-tetrahydro-2H-pyran-2-one;
6(R)-[2-[8(S)-(2,2-dimethylbutyryloxy)-2(S)-methyl-5(R)-hydroxy-6(R)-methyl-1,2,3,4,4a(R),5,6,7,8,8a(R)-decahydronaphthyl-1(S)]ethyl]-4(R)-hydroxy-3,4,5,6-tetrahydro-2H-pyran-2-one;
6(R)-[2-[8(S)-(2,2-dimethylbutyryloxy)-2(S)-methyl-5(R)-hydroxy-6(R)-methyl-1,2,4a(R),5,6,7,8,8a(R)-octahydronaphthyl-1(S)]ethyl]-4(R)-hydroxy-3,4,5,6-tetrahydro-2H-pyran-2-one;
6(R)-[8(S)-(2,2-dimethylbutyryloxy)-2(S)-methyl-5-oxo-6(R)-methyl-1,2,3,4,4a(R),6,7,8,8a(R)-nonahydronaphthyl-1(S)]ethyl]-4(R)-hydroxy-3,4,5,6-tetrahydro-2H-pyran-2-one;
6(R)-[2-[8(S)-(2,2-dimethylbutyryloxy)-2(S)-methyl-5-oxo-6(S)-methyl-1,2,3,4,4a(R),6,7,8,8a(R)-nonahydronaphthyl-1(S)]ethyl]-4(R)-hydroxy-3,4,5,6-tetrahydro-2H-pyran-2-one; and the corresponding ring opened dihydroxy acid and esters thereof.

**8.** A hypocholesterolemic, hypolipidemic pharmaceutical composition comprising a pharmaceutically acceptable carrier and a therapeutically effective amount of a compound as defined in Claim 1.

**9.** A composition of Claim 8 in which the compound is selected from:
6(R)-[2-[8(S)-(2,2-dimethylbutyryloxy)-2(S)-methyl-5(R)-hydroxy-6(R)-methyl-1,2,3,4,4a(R),5,6,7,8,8a(R)-decahydronaphthyl-1(S)]ethyl]-4(R)-hydroxy-3,4,5,6-tetrahydro-2H-pyran-2-one;
6(R)-[2-[8(S)-(2,2-dimethylbutyryloxy)-2(S)-methyl-5(S)-hydroxy-6(S)-methyl-1,2,3,4,4a(R),5,6,7,8,8a(R)-decahydronaphthyl-1(S)]ethyl]-4(R)-hydroxy-3,4,5,6-tetrahydro-2H-pyran-2-one;
6(R)-[2-[8(S)-(2,2-dimethylbutyryloxy)-2(S)-methyl-5(R)-benzylaminocarbonyloxy-6(R)-methyl-1,2,3,4,4a-(R),5,6,7,8,8a(R)-decahydronaphthyl-1(S)]ethyl]-4(R)-hydroxy-3,4,5,6-tetrahydro-2H-pyran-2-one;
6(R)-[2-[8(S)-(2,2-dimethylbutyryloxy)-2(S)-methyl-5(R)-diphenylphosphinyloxy-6(R)-methyl-1,2,3,4,4a-(R),5,6,7,8,8a(R)-decahydronaphthyl-1(S)]ethyl]-4(R)-hydroxy-3,4,5,6-tetrahydro-2H-pyran-2-one;
6(R)-[2-[8(S)-(2,2-dimethylbutyryloxy)-2(S)-methyl-5(R)-(1-phenylethyl-2-oxy)-6(R)-methyl-1,2,3,4,4a(R)-,5,6,7,8,8a(R)-decahydronaphthyl-1(S)]ethyl]-4(R)-hydroxy-3,4,5,6-tetrahydro-2H-pyran-2-one;
6(R)-[2-[8(S)-(2,2-dimethylbutyryloxy)-2(S)-methyl-5(R)-dibenzylaminocarbonyloxy-6(R)-methyl-1,2,3,4,4a(R),5,6,7,8,8a(R)-decahydronaphthyl-1(S)]ethyl]-4(R)-hydroxy-3,4,5,6-tetrahydro-2H-pyran-2-one;
6(R)-[2-[8(S)-(2,2-dimethylbutyryloxy)-2(S)-methyl-5-oxo-6(R)-methyl-6(R)-methyl-1,2,3,4,4a(R)-,5,6,7,8,8a(R)-nonahydronaphthyl-1(S)]ethyl]-4(R)-hydroxy-3,4,5,6-tetrahydro-2H-pyran-2-one;
6(R)-[2-[8(S)-(2,2-dimethylbutyryloxy)-2(S)-methyl-5-oxo-6(S)-methyl-1,2,3,4,4a(R),5,6,7,8,8a(R)-nonahydronaphthyl-1(S)]ethyl]-4(R)-hydroxy-3,4,5,6-tetrahydro-2H-pyran-2-one;
6(R)-{2-{8(S)-(2,2-dimethylbutyryloxy)-2(S)-methyl-5(R)-hydroxy-6(S)-methyl-1,2,3,4a(R),6,7,8,8a(R)-decahydronaphthyl-1(S)}ethyl}-4(R)-hydroxy-3,4,5,6-tetrahydro-2H-pyran-2-one;
6(R)-[2-[8(S)-(2,2-dimethylbutyryloxy)-2(S)-methyl-5(R)-hydroxy-6(R)-methyl-1,2,4a(R),5,6,7,8,8a(R)-octahydronaphthyl-1(S)]ethyl]-4(R)-hydroxy-3,4,5,6-tetrahydro-2H-pyran-2-one; and the corresponding ring opened dihydroxy acid and esters.

**10.** A process for the formation of a compound (1-6):

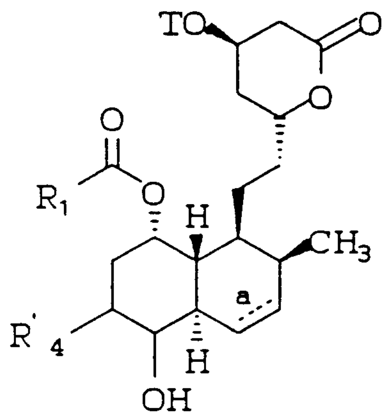

(1-6)

wherein:

$R_1$ is selected from:

(1) $C_{1-10}$ alkyl;
(2) substituted $C_{1-10}$ alkyl in which one or more substituent(s) is selected from
  (a) halogen,
  (b) hydroxy,
  (c) $C_{1-10}$ alkoxy,
  (d) $C_{1-5}$ alkoxycarbonyl,
  (e) $C_{1-5}$ acyloxy,
  (f) $C_{3-8}$ cycloalkyl,
  (g) phenyl,
  (h) substituted phenyl in which the substituents are X and Y,
  (i) $C_{1-10}$ alkylS(0)$_n$ in which n is 0 to 2,
  (j) $C_{3-8}$ cycloalkylS(0)$_n$,
  (k) phenylS(0)$_n$,
  (l) substituted phenylS(0)$_n$ in which the substituents are X and Y, and
  (m) oxo;
(3) $C_{1-10}$ alkoxy;
(4) $C_{2-10}$ alkenyl;
(5) $C_{3-8}$ cycloalkyl;
(6) substituted $C_{3-8}$ cycloalkyl in which one substituent is selected from
  (a) $C_{1-10}$ alkyl
  (b) substituted $C_{1-10}$ alkyl in which the substituent is selected from
    (i) halogen,
    (ii) hydroxy,
    (iii) $C_{1-10}$ alkoxy,
    (iv) $C_{1-5}$ alkoxycarbonyl,
    (v) $C_{1-5}$ acyloxy,
    (vi) phenyl,
    (vii) substituted phenyl in which the substituents are X and Y
    (viii) $C_{1-10}$ alkylS(O)$_n$,
    (ix) $C_{3-8}$ cycloalkylS(O)$_n$,
    (x) phenylS(O)$_n$,
    (xi) substituted phenylS(O)$_n$ in which the substituents are X and Y, and
    (xii) oxo,

(c) $C_{1-10}$ alkylS(0)$_n$,
(d) $C_{3-8}$ cycloalkylS(0)$_n$,
(e) phenylS(0)$_n$,
(f) substituted phenylS(0)$_n$ in which the substituents are X and Y,
(g) halogen,
(h) hydroxy,
(i) $C_{1-10}$ alkoxy,
(j) $C_{1-5}$ alkoxycarbonyl,
(k) $C_{1-5}$ acyloxy,
(l) phenyl, and
(m) substituted phenyl in which the substituents are X and Y;
(7) phenyl;
(8) substituted phenyl in which the substituents are X and Y;
(9) amino;
(10) $C_{1-5}$ alkylamino;
(11) di($C_{1-5}$ alkyl)amino;
(12) phenylamino;
(13) substituted phenylamino in which the substituents are X and Y;
(14) phenyl $C_{1-10}$ alkylamino;
(15) substituted phenyl $C_{1-10}$ alkylamino in which the substituents are X and Y;
(16) a member selected from
(a) piperidinyl,
(b) pyrrolidinyl,
(c) piperazinyl,
(d) morpholinyl, and
(e) thiomorpholinyl; and
(17) $R_{10}$S in which $R_{10}$ is selected from
(a) $C_{1-10}$ alkyl,
(b) phenyl, and
(c) substituted phenyl in which the substituents are X and Y;

$R'_4$ is;
(1) halogen;
(2) $C_{1-10}$ alkyl; and
(3) substituted $C_{1-10}$ alkyl in which one or more substituents is selected from
(a) hydrogen,
(b) OT,
(c) $C_{1-10}$ alkoxy
(d) $C_{1-5}$ alkoxycarbonyl,
(e) $C_{1-5}$ alkylacyloxy,
(f) phenylacyloxy,
(g) phenoxycarbonyl,
(h) phenyl $C_{1-5}$ alkylacyloxy,
(i) phenyl $C_{1-5}$ alkoxy,
(j) amino,
(k) $C_{1-5}$ alkylamino,
(l) di($C_{1-5}$ alkyl)amino,
(m) phenylamino,
(n) substituted phenylamino in which the substituents are X and Y;
(o) phenyl $C_{1-5}$ alkylamino,
(p) substituted phenyl $C_{1-5}$ alkylamino in which the substituents are X and Y,
(q) $C_{3-8}$ cycloalkyl,
(r) phenyl,
(s) substituted phenyl in which the substituents are X and Y,
(t) phenylS(O)$_n$,
(u) substituted phenyl S(O)$_n$ in which the substituents are X and Y,

(v) phenyl $C_{1-5}$ alkyl $S(O)_n$,
(w) $C_{1-5}$ alkyl$S(O)_n$;
(x) phenylaminoacyloxy,
(y) $C_{1-5}$alkylaminoacyloxy,
(z) $C_{1-5}$alkylacylamino,
(aa) di(phenyl$C_{1-5}$alkyl)phosphonyl
(bb) di($C_{1-5}$alkyl)phosphinyl

(4) $R'_4$ together with the carbon atom to which it is attached represents a $C_{3-8}$ carbocyclic ring;

X and Y are independently selected from:
a) OH;
b) halogen,
c) trifluoromethyl,
d) $C_{1-3}$alkoxy,
e) $C_{1-3}$alkylocarbonyloxy,
f) phenylcarbonyloxy,
g) $C_{1-3}$alkoxycarbonyl,
h) phenyloxycarbonyl,
i) hydrogen,
j) $C_{1-5}$alkyl;

T is H, tert-butyldimethylsilyl, tert-butyl diphenylsilyl, trimethylsilyl, triethylsilyl, triisopropylsilyl, or tetrahydropyranyl;

halogen is Cl or F;
a is a single bond or a double bond; which comprises treatment of a compound

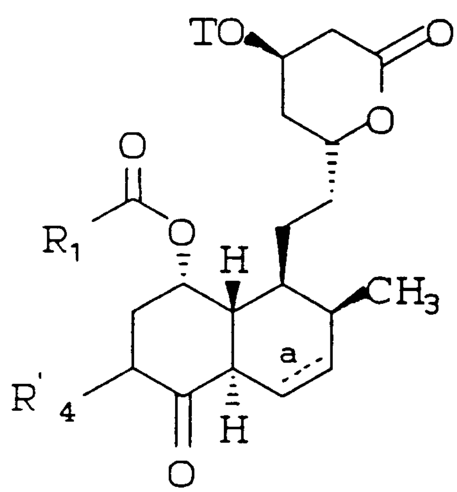

(1-5)

with sodium borohydride to form compound (1-6); and optionally where T is not H, removal of the hydroxyl protecting group by treatment with tetrabutyl ammonium fluoride and acetic acid to form a compound (1-6)

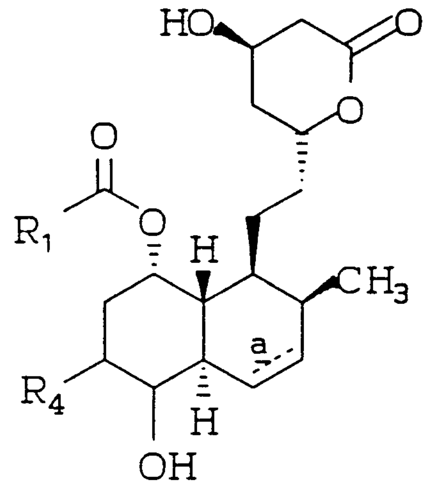

(1-6)

wherein :

$R_4$ is $R'_4$ where T is H.

**11.** The use of a compound of claim 1 for the manufacture of a medicament for treating arteriosclerosis, familial hypercholesterolemia or hyperlipidemia.

## Patentansprüche

**1.** Verbindung, dargestellt durch die Strukturformeln (I) oder (II):

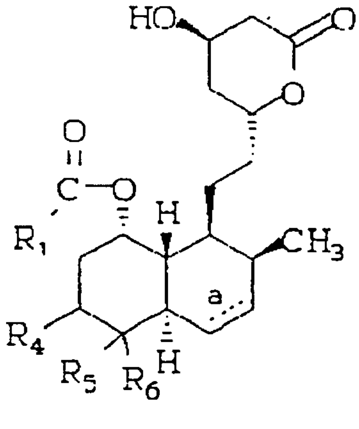

(I)

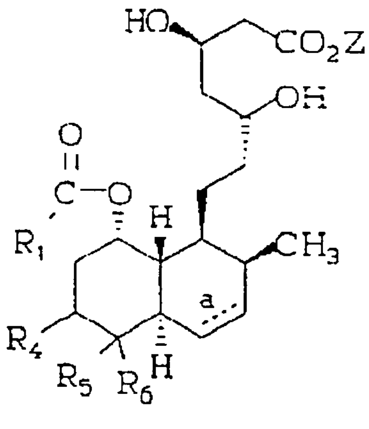

(II)

worin:

$R_1$ ausgewählt ist aus:

(1) $C_{1-10}$-Alkyl;

(2) substituiertem $C_{1-10}$-Alkyl, in dem ein oder mehrere Substituent(en) ausgewählt sind aus

(a) Halogen,
(b) Hydroxy,
(c) $C_{1-10}$-Alkoxy,
(d) $C_{1-5}$-Alkoxycarbonyl,
(e) $C_{1-5}$-Acyloxy,
(f) $C_{3-8}$-Cycloalkyl,
(g) Phenyl,
(h) substituiertem Phenyl, in dem die Substituenten X und Y sind,
(i) $C_{1-10}$-AlkylS(O)$_n$, in dem n 0 bis 2 bedeutet,
(j) $C_{3-8}$-CycloalkylS(O)$_n$,
(k) PhenylS(O)$_n$,
(l) substituiertem PhenylS(O)$_n$, worin die Substituenten X und Y bedeuteten, und
(m) oxo;

(3) $C_{1-10}$-Alkoxy;
(4) $C_{2-10}$-Alkenyl;
(5) $C_{3-8}$-Cycloalkyl;
(6) substituiertem $C_{3-8}$-Cycloalkyl, in dem ein Substituent ausgewählt ist aus

(a) $C_{1-10}$-Alkyl,
(b) substituiertem $C_{1-10}$-Alkyl, in dem der Substituent ausgewählt ist aus

(i) Halogen,
(ii) Hydroxy,
(iii) $C_{1-10}$-Alkoxy,
(iv) $C_{1-5}$-Alkoxycarbonyl,
(v) $C_{1-5}$-Acyloxy,
(vi) Phenyl,
(vii) substituiertem Phenyl, in dem die Substituenten X und Y bedeuten,
(viii) $C_{1-10}$-AlkylS(O)$_n$,
(ix) $C_{3-8}$-CycloalkylS(O)$_n$,
(x) PhenylS(O)$_n$,
(xi) substituiertem PhenylS(O)$_n$, in dem die Substituenten X und Y sind,
(xii) Oxo,

(c) $C_{1-10}$-AlkylS(O)$_n$,
(d) $C_{3-8}$-CycloalkylS(O)$_n$,
(e) PhenylS(O)$_n$,
(f) substituiertem PhenylS(O)$_n$, in dem die Substituenten X und Y sind,
(g) Halogen,
(h) Hydroxy,
(i) $C_{1-10}$-Alkoxy,
(j) $C_{1-5}$-Alkoxycarbonyl,
(k) $C_{1-5}$-Acyloxy,
(l) Phenyl, und
(m) substituiertem Phenyl, in dem die Substituenten X und Y sind;

(7) Phenyl;
(8) substituiertem Phenyl, in dem die Substituenten X und Y sind;
(9) Amino;
(10) $C_{1-5}$-Alkylamino;
(11) Di($C_{1-5}$-alkyl)-amino;
(12) Phenylamino;
(13) substituiertem Phenylamino, in dem die Substituenten X und Y sind;
(14) Phenyl-$C_{1-10}$-alkylamino;

(15) substituiertem Phenyl$C_{1-10}$-alkylamino, in dem die Substituenten X und Y sind;
(16) einem Glied, ausgewählt aus
(a) Piperidinyl,
(b) Pyrrolidinyl,
(c) Piperazinyl,
(d) Morpholinyl, und
(e) Thiomorpholinyl; und
(17) $R_{10}S$, in dem $R_{10}$ ausgewählt ist aus
(a) $C_{1-10}$-Alkyl,
(b) Phenyl, und
(c) substituiertem Phenyl, in dem die Substituenten X und Y sind;

$R_4$ für folgendes steht:
(1) Wasserstoff;
(2) $C_{1-10}$-Alkyl; und
(3) substituiertes $C_{1-10}$-Alkyl, in dem ein oder mehreren Substituenten ausgewählt sind aus
(a) Halogen,
(b) Hydroxy,
(c) $C_{1-10}$-Alkoxy,
(d) $C_{1-5}$-Alkoxycarbonyl,
(e) $C_{1-5}$-Alkylacyloxy;
(f) Phenylacyloxy,
(g) Phenoxycarbonyl,
(h) Phenyl-$C_{1-5}$-alkylacyloxy,
(i) Phenyl-$C_{1-5}$-alkoxy,
(j) Amino,
(k) $C_{1-5}$-Alkylamino,
(l) Di($C_{1-5}$-alkyl)-amino,
(m) Phenylamino,
(n) substituiertem Phenylamino, in dem die Substituenten X und Y sind,
(o) Phenyl-$C_{1-5}$-alkylamino,
(p) substituiertem Phenyl-$C_{1-5}$-alkylamino, in dem die Substituenten X und Y sind,
(q) $C_{3-8}$-Cycloalkyl,
(r) Phenyl,
(s) substituiertem Phenyl, in dem die Substituenten X und Y sind,
(t) Phenyl$S(O)_n$,
(u) substituiertem Phenyl$S(O)_n$, in dem die Substituenten X und Y sind,
(v) Phenyl-$C_{1-5}$-alkyl-$S(O)_n$,
(w) $C_{1-5}$-Alkyl$S(O)_n$,
(x) Phenylaminoacyloxy,
(y) $C_{1-5}$-Alkylaminoacyloxy,
(z) $C_{1-5}$-Alkylacylamino,
(aa) Di(phenyl-$C_{1-5}$-alkyl)-phosphonyl,
(bb) Di($C_{1-5}$-alkyl)-phosphinyl,
(4) $R_4$ zusammen mit dem Kohlenstoffatom, an das es angeknüpft ist, einen carbocyclischen $C_{3-8}$-Ring darstellt;

$R_5$ und $R_6$ unabhängig für H, OH, $OR_7$ stehen oder $R_5$ und $R_6$ zusammen mit dem Kohlenstoffatom, an das sie angeknüpft sind, C=O darstellen oder $R_5$ und $R_6$ zusammen mit dem Kohlenstoffatom, an das sie angeknüpft sind, einen carbocylischen Ring mit 3 bis 7 Atomen darstellen, mit der Maßgabe, daß wenn $R_5$ für H steht, $R_6$ OH oder $OR_7$ bedeutet und wenn $R_5$ für OH steht, $R_6$ H bedeutet und wenn $R_5$ für $OR_7$ steht, $R_6$ H bedeutet;

$R_7$ für

$$-\overset{\overset{O}{\|}}{P}-R_8R_9,\quad -\overset{\overset{O}{\|}}{C}NR_8R_9 \text{ oder } -\overset{\overset{O}{\|}}{C}-R_8-,\quad -\overset{\overset{O}{\|}}{C}-O-R_8,$$

Phenyl-$C_{1-3}$-alkyl, $C_{1-5}$-Alkyl steht;

$R_8$ und $R_9$ unabhängig für H, $C_{1-3}$-Alkyl, Phenyl-$C_{1-3}$-alkyl oder Aryl stehen, worin Aryl für Phenyl, Naphthyl, Pyridyl, Furanyl, Thienyl oder Phenyl, Naphthyl, Pyridyl, Furanyl oder Thienyl, substituiert mit X- und Y-Gruppen, steht, mit der Maßgabe, daß wenn $R_7$ für

$$-\overset{\overset{O}{\|}}{C}-O-R_8$$

steht, $R_8$ nicht H bedeutet, und wenn $R_7$ für

$$-\overset{\overset{O}{\|}}{P}-R_8R_9,$$

weder $R_8$ noch $R_9$ H bedeuten;

X und Y unabhängig ausgewählt sind aus:

a) OH;
b) Halogen;
c) Trifluormethyl;
d) $C_{1-3}$-Alkoxy;
e) $C_{1-3}$-Alkylcarbonyloxy;
f) Phenylcarbonyloxy;
g) $C_{1-3}$-Alkoxycarbonyl;
h) Phenyloxycarbonyl;
i) Wasserstoff;
j) $C_{1-5}$-Alkyl;

Z ausgewählt ist aus:

(1) Wasserstoff;
(2) $C_{1-5}$-Alkyl;
(3) substituiertem $C_{1-5}$-Alkyl, worin die Substituenten ausgewählt sind aus:
  (a) Phenyl,
  (d) Dimethylamino, und
  (c) Acetylamino, und
(4) 2,3-Dihydroxypropyl;

Halogen Cl oder F bedeutet,
a für eine Einfach- oder Doppelbindung steht, mit der Maßgabe, daß, wenn a eine Doppelbindung ist und $R_5$ oder $R_6$ OH bedeutet, die Konfiguration in Stellung 5 des Naphthylrings 5(R) ist;
oder ein pharmazeutisch verträgliches Salz davon.

**2.** Verbindung nach Anspruch 1, worin
$R_4$

(1) Wasserstoff;
(2) $C_{1-10}$-Alkyl;
(3) substituiertes $C_{1-10}$-Alkyl, in dem ein oder mehrere Substituenten ausgewählt sind aus:
  (a) Halogen,
  (b) Hydroxy,
  (c) Amino;
(4) $CH_2R_{12}$, in dem $R_{12}$ ausgewählt ist aus:
  (a) $C_{1-5}$-Alkoxy,

(b) $C_{1-5}$-Alkoxycarbonyl,
(c) $C_{1-5}$-Alkylacyloxy;
(d) Phenylacyloxy,
(e) Phenoxycarbonyl,
(f) Phenyl-$C_{1-5}$-alkyl,
(g) Phenyl-$C_{1-5}$-alkoxy,
(h) $C_{1-5}$-Alkylamino,
(i) Di($C_{1-5}$-alkyl)-amino,
(j) Phenylamino,
(k) substituiertem Phenylamino, in dem die Substituenten X und Y sind,
(l) Phenyl-$C_{1-5}$-alkylamino,
(m) substituiertem Phenyl-$C_{1-5}$-alkylamino, in dem die Substituenten X und Y sind,
(n) $C_{3-8}$-Cycloalkyl,
(o) Phenyl,
(p) substituiertem Phenyl, in dem die Substituenten X und Y sind,
(q) Phenyl$S(O)_n$,
(r) substituiertem Phenyl$S(O)_n$, in dem die Substituenten X und Y sind,
(s) Phenyl-$C_{1-5}$-alkyl-$S(O)_n$,
(t) $C_{1-5}$-Alkyl$S(O)_n$,
(u) Phenylaminoacyloxy,
(v) $C_{1-5}$-Alkylaminoacyloxy,
(w) $C_{1-5}$-Alkylacylamino,
(x) Di(phenyl-$C_{1-5}$-alkyl)-phosphonyl,
(y) Di($C_{1-5}$-alkyl)-phosphinyl;

(5) $R_4$ zusammen mit dem Kohlenstoffatom, an das es angeknüpft ist, einen Cyclopropanring darstellt;

$R_5$ und $R_6$ unabhängig für H, OH, $OR_7$ stehen oder $R_5$ und $R_6$ zusammen mit dem Kohlenstoff, an das sie angeknüpft sind, C=O darstellen oder $R_5$ und $R_6$ zusammen mit dem Kohlenstoff, an das sie angeknüpft sind, einen Cyclopropanring darstellen, mit der Maßgabe, daß wenn $R_5$ für H steht, $R_6$ OH oder $OR_7$ bedeutet und wenn $R_5$ für OH steht, $R_6$ H bedeutet und wenn $R_5$ für $OR_7$, $R_6$ H bedeutet;

X und Y unabhängig ausgewählt werden aus:
a) OH,
b) F,
c) Trifluormethyl,
d) $C_{1-3}$-Alkoxy,
e) Wasserstoff,
f) $C_{1-5}$-Alkyl.

**3.** Verbindung nach Anspruch 2, worin:
$R_1$ für $C_{1-10}$-Alkyl steht; und
$R_4$ für $CH_3$, H oder $CH_2$-Phenyl steht;
$R_7$ für

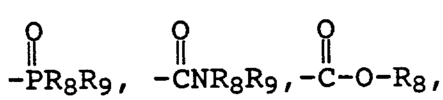

$C_{1-5}$-Alkyl oder Phenyl-$C_{1-3}$-alkyl steht;
$R_8$ und $R_9$ unabhängig für H, $C_{1-3}$-Alkyl, Phenyl-$C_{1-3}$-alkyl oder Aryl stehen, worin Aryl für Phenyl oder Naphthyl oder Phenyl oder Naphthyl, substituiert mit X, steht.

**4.** Verbindung nach Anspruch 3 worin:
$R_1$ für 2-Butyl oder 2-Methyl-2-butyl steht;
$R_4$ für $CH_3$ steht.

**5.** Verbindung nach Anspruch 2, worin:
$R_1$ für $C_{1-10}$-Alkyl steht;
$R_4$ für $CH_2OH$ oder Phenylacyloxymethyl steht;
$R_7$ für

$$-\overset{O}{\overset{\|}{P}}R_8R_9,\quad -\overset{O}{\overset{\|}{C}}NR_8R_9,$$

$C_{1-5}$-Alkyl oder Phenyl-$C_{1-5}$-alkyl steht;
$R_8$ und $R_9$ unabhängig für H, $C_{1-3}$-Alkyl, Phenyl-$C_{1-3}$-alkyl oder Aryl stehen, worin Aryl für Phenyl oder Naphthyl oder Phenyl oder Naphthyl, substituiert mit X, steht.

**6.** Verbindung nach Anspruch 5, worin:
$R_1$ für 2-Butyl oder 2-Methyl-2-butyl steht;
$R_4$ für $CH_2OH$ steht.

**7.** Verbindung nach Anspruch 4, ausgewählt aus:
6(R)-[2-[8(S)-(2,2-Dimethylbutyryloxy)-2(S)-methyl-5(R)-hydroxy-6(R)-methyl-1,2,3,4,4a(R),5,6,7,8,8a(R)-decahydronaphthyl-1(S)]-ethyl]-4(R)-hydroxy-3,4,5,6-tetrahydro-2H-pyran-2-on;
6(R)-[2-[8(S)-(2,2-Dimethylbutyryloxy)-2(S)-methyl-5(S)-hydroxy-6(S)-methyl-1,2,3,4,4a(R),5,6,7,8,8a(R)-decahydronaphthyl-1(S)]-ethyl]-4(R)-hydroxy-3,4,5,6-tetrahydro-2H-pyran-2-on;
6(R)-[2-[8(S)-(2,2-Dimethylbutyryloxy)-2(S)-methyl-5(R)-benzylaminocarbonyloxy-6(R)-methyl-1,2,3,4,4a-(R),5,6,7,8,8a(R)-decahydronaphthyl-1(S)]-ethyl]-4(R)-hydroxy-3,4,5,6-tetrahydro-2H-pyran-2-on;
6(R)-[2-[8(S)-(2,2-Simethylbutyryloxy)-2(S)-methyl-5(R)-diphenylphosphinyloxy-6(R)-methyl-1,2,3,4,4a-(R),5,6,7,8,8a(R)-decahydronaphthyl-1(S)]-ethyl]-4(R)-hydroxy-3,4,5,6-tetrahydro-2H-pyran-2-on;
6(R)-[2-[8(S)-(2,2-Dimethylbutyryloxy)-2(S)-methyl-5(R)-(1-phenylethyl-2-oxy)-6(R)-methyl-1,2,3,4,4a(R)-,5,6,7,8,8a(R)-decahydronaphthyl-1(S)-{ethyl}-4(R)-hydroxy-3,4,5,6-tetrahydro-2H-pyran-2-on;
6(R)-[2-[8(S)-(2,2-Dimethylbutyryloxy)-2(S)-methyl-5(R)-dibenzylaminocarbonyloxy-6(S)-methyl-1,2,3,4,4a(R),5,6,7,8,8a(R)-decahydronaphthyl-1(S)]-ethyl]-4(R)-hydroxy-3,4,5,6-tetrahydro-2H-pyran-2-on;
6(R)-[2-[8(S)-(2,2-Dimethylbutyryloxy)-2(S)-methyl-5(R)-hydroxy-6(S)-methyl-1,2,3,4,4a(R),5,6,7,8,8a(R)-decahydronaphthyl-1(S)]-ethyl]-4(R)-hydroxy-3,4,5,6-tetrahydro-2H-pyran-2-on;
6(R)-[8(S)-(2,2-Dimethylbutyryloxy)-2(S)-methyl-5(R)-hydroxy-6(R)-methyl-1,2, 4a(R),5,6,7,8,8a(R)-octahydronaphthyl-1(S)]-ethyl]-4(R)-hydroxy-3,4,5,6-tetrahydro-2H-pyran-2-on;
6(R)-[2-[8(S)-(2,2-Dimethylbutyryloxy)-2(S)-methyl-5-oxo-6(R)-methyl-1,2,3,4,4a(R),6,7,8,8a(R)-nonahydronaphthyl-1(S)]-ethyl]-4(R)-hydroxy-3,4,5,6-tetrahydro-2H-pyran-2-on;
6(R)-[2-[8(S)-(2,2-Dimethylbutyryloxy)-2(S)-methyl-5-oxo-6(S)-methyl-1,2,3,4,4a(R),6,7,8,8a(R)-nonahydronaphthyl-1(S)]-ethyl]-4(R)-hydroxy-3,4,5,6-tetrahydro-2H-pyran-2-on; und die entsprechende ringeöffnete Dihydroxysäure und die Ester davon.

**8.** Hypocholesterinämische, hypolipidämische pharmazeutische Zusammensetzung, umfassend einen pharmazeutisch vertäglichen Träger und eine pharmazeutische wirksame Menge einer Verbindung nach Anspruch 1.

**9.** Zusammensetzung nach Anspruch 8, in der die Verbindung ausgewählt ist aus:
6(R)-[2-[8(S)-(2,2-Dimethylbutyryloxy)-2(S)-methyl-5(R)-hydroxy-6(R)-methyl-1,2,3,4,4a(R),5,6,7,8,8a(R)-decahydronaphthyl-1(S)]-ethyl]-4(R)-hydroxy-3,4,5,6-tetrahydro-2H-pyran-2-on;
6(R)-[2-[8(S)-(2,2-Dimethylbutyryloxy)-2(S)-methyl-5(S)-hydroxy-6(S)-methyl-1,2,3,4,4a(R),5,6,7,8,8a(R)-decahydronaphthyl-1(S)]-ethyl]-4(R)-hydroxy-3,4,5,6-tetrahydro-2H-pyran-2-on;
6(R)-[2-[8(S)-(2,2-Dimethylbutyryloxy)-2(S)-methyl-5(R)-benzylaminocarbonyloxy-6(R)-methyl-1,2,3,4,4a-(R),5,6,7,8,8a(R)-decahydronaphthyl-1(S)]-ethyl]-4(R)-hydroxy-3,4,5,6-tetrahydro-2H-pyran-2-on;
6(R)-[2-[8(S)-(2,2-Dimethylbutyryloxy)-2(S)-methyl-5(R)-diphenylphosphinyloxy-6(R)-methyl-1,2,3,4,4a-(R),5,6,7,8,8a(R)-decahydronaphthyl-1(S)]-ethyl]-4(R)-hydroxy-3,4,5,6-tetrahydro-2H-pyran-2-on;
6(R)-[2-[8(S)-(2,2-Dimethylbutyryloxy)-2(S)-methyl-5(R)-(1-phenylethyl-2-oxy)-6(R)-methyl-1,2,3,4,4a(R)-,5,6,7,8,8a(R)-decahydronaphthyl-1(S)]-ethyl]-4(R)-hydroxy-3,4,5,6-tetrahydro-2H-pyran-2-on;
6(R)-[2-[8(S)-(2,2-Dimethylbutyryloxy)-2(S)-methyl-5(R)-dibenzylaminocarbonyloxy-6(R)-methyl-1,2,3,4,4a(R),5,6,7,8,8a(R)-decahydronaphthyl-1(S)]-ethyl]-4(R)-hydroxy-3,4,5,6-tetrahydro-2H-pyran-2-on;
6(R)-[2-[8(S)-(2,2-Dimethylbutyryloxy)-2(S)-methyl-5-oxo-6(R)-methyl-6(R)-methyl-1,2,3,4,4a(R)-,5,6,7,8,8a(R)-nonahydronaphthyl-1(S)]-ethyl]-4(R)-hydroxy-3,4,5,6-tetrahydro-2H-pyran-2-on;
6(R)-[8(S)-(2,2-Dimethylbutyryloxy)-2(S)-methyl-5-oxo-6(S)-methyl-1,2,3,4,4a(R),6,7,8,8a(R)-nonahydronaphthyl-1(S)]-ethyl]-4(R)-hydroxy-3,4,5,6-tetrahydro-2H-pyran-2-on;
6(R)-{2-{8(S)-(2,2-Dimethylbutyryloxy)-2(S)-methyl-5(R)-hydroxy-6(S)-methyl-1,2,3,4,4a(R),6,7,8,8a(R)-decahydronaphthyl-1(S)}-ethyl}-4(R)-hydroxy-3,4,5,6-tetrahydro-2H-pyran-2-on;
6(R)-[8(S)-(2,2-Dimethylbutyryloxy)-2(S)-methyl-5(R)-hydroxy-6(R)-methyl-1,2,4a(R),5,6,7,8,8a(R)-octahydronaphthyl-1(S)]-ethyl]-4(R)-hydroxy-3,4,5,6-tetrahydro-2H-pyran-2-on; und die entsprechende ringeöffnete Dihydroxysäure und die Ester davon.

**10.** Verfahren zur Bildung einer Verbindung (1-6):

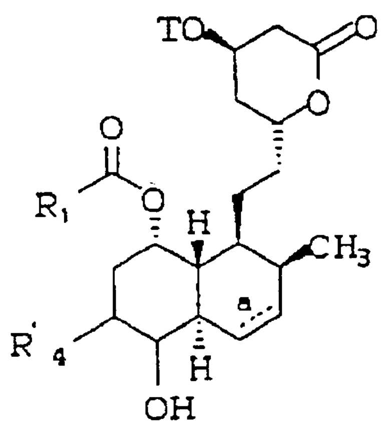

(1-6)

$R_1$ ausgewählt ist aus:

(1) $C_{1-10}$-Alkyl;
(2) substituiertem $C_{1-10}$-Alkyl, in dem ein oder mehreren Substituent(en) ausgewählt sind aus
  (a) Halogen,
  (b) Hydroxy,
  (c) $C_{1-10}$-Alkoxy,
  (d) $C_{1-5}$-Alkoxycarbonyl,
  (e) $C_{1-5}$-Acyloxy,
  (f) $C_{3-8}$-Cycloalkyl,
  (g) Phenyl,
  (h) substituiertem Phenyl, in dem die Substituenten X und Y sind,
  (i) $C_{1-10}$-AlkylS(O)$_n$, in dem n 0 bis 2 bedeutet,
  (j) $C_{3-8}$-CycloalkylS(O)$_n$,
  (k) PhenylS(O)$_n$,
  (l) substituiertem PhenylS(O)$_n$, in dem die Substituenten X und Y bedeuteten, und
  (m) oxo;
(3) $C_{1-10}$-Alkoxy;
(4) $C_{2-10}$-Alkenyl;
(5) $C_{3-8}$-Cycloalkyl;
(6) substituiertem $C_{3-8}$-Cycloalkyl, in dem ein Substituent ausgewählt ist aus
  (a) $C_{1-10}$-Alkyl,
  (b) substituiertem $C_{1-10}$-Alkyl, in dem der Substituent ausgewählt ist aus

(i) Halogen,
(ii) Hydroxy,
(iii) $C_{1-10}$-Alkoxy,
(iv) $C_{1-5}$-Alkoxycarbonyl,
(v) $C_{1-5}$-Acyloxy,
(vi) Phenyl,
(vii) substituiertem Phenyl, in dem die Substituenten X und Y sind,
(viii) $C_{1-10}$-AlkylS(O)$_n$,
(ix) $C_{3-8}$-CycloalkylS(O)$_n$,
(x) PhenylS(O)$_n$,
(xi) substituiertem PhenylS(O)$_n$, in dem die Substituenten X und Y sind, und
(xii) Oxo,
(c) $C_{1-10}$-AlkylS(O)$_n$,
(d) $C_{3-8}$-CycloalkylS(O)$_n$,
(e) PhenylS(O)$_n$,
(f) substituiertem PhenylS(O)$_n$, in dem die Substituenten X und Y sind,
(g) Halogen,
(h) Hydroxy,
(i) $C_{1-10}$-Alkoxy,
(j) $C_{1-5}$-Alkoxycarbonyl,
(k) $C_{1-5}$-Acyloxy,
(l) Phenyl, und
(m) substituiertem Phenyl, in dem die Substituenten X und Y sind;
(7) Phenyl;
(8) substituiertem Phenyl, in dem die Substituenten X und Y sind;
(9) Amino;
(10) $C_{1-5}$-Alkylamino;
(11) Di($C_{1-5}$-alkyl)-amino;
(12) Phenylamino;
(13) substituiertem Phenylamino, in dem die Substituenten X und Y sind;
(14) Phenyl-$C_{1-10}$-alkylamino;
(15) substituiertem Phenyl$C_{1-10}$-alkylamino, in dem die Substituenten X und Y sind;
(16) einem Glied, ausgewählt aus
(a) Piperidinyl,
(b) Pyrrolidinyl,
(c) Piperazinyl,
(d) Morpholinyl, und
(e) Thiomorpholinyl; und
(17) $R_{10}$S, in dem $R_{10}$ ausgewählt ist aus
(a) $C_{1-10}$-Alkyl,
(b) Phenyl, und
(c) substituiertem Phenyl, in dem die Substituenten X und Y sind;

$R'_4$ für folgendes steht:
(1) Wasserstoff;
(2) $C_{1-10}$-Alkyl; und
(3) substituiertes $C_{1-10}$-Alkyl, in dem ein oder mehreren Substituenten ausgewählt sind aus
(a) Halogen,
(b) OT,
(c) $C_{1-10}$-Alkoxy,
(d) $C_{1-5}$-Alkoxycarbonyl,
(e) $C_{1-5}$-Alkylacyloxy;
(f) Phenylacyloxy,

(g) Phenoxycarbonyl,
(h) Phenyl-$C_{1-5}$-alkylacyloxy,
(i) Phenyl-$C_{1-5}$-alkoxy,
(j) Amino,
(k) $C_{1-5}$-Alkylamino,
(l) Di ($C_{1-5}$)-amino,
(m) Phenylamino,
(n) substituiertem Phenylamino, in dem die Substituenten X und Y sind,
(o) Phenyl-$C_{1-5}$-alkylamino,
(p) substituiertem Phenyl-$C_{1-5}$-alkylamino, in dem die Substituenten X und Y sind,
(q) $C_{3-8}$-cycloalkyl,
(r) Phenyl,
(s) substituiertem Phenyl, in dem die Substituenten X und Y sind,
(t) Phenyl$S(O)_n$,
(u) substituiertem Phenyl$S(O)_n$, in dem die Substituenten X und Y sind,
(v) Phenyl-$C_{1-5}$-alkyl$S(O)_n$,
(w) $C_{1-5}$-Alkyl$S(O)_n$,
(x) Phenylaminoacyloxy,
(y) $C_{1-5}$-Alkylaminoacyloxy,
(z) $C_{1-5}$-Alkylacylamino,
(aa) Di(phenyl-$C_{1-5}$-alkyl)-phosphonyl,
(bb) Di($C_{1-5}$-alkyl)-phosphinyl,

(4) $R'_4$ zusammen mit dem Kohlenstoffatom, an das es angeknüpft ist, einen carbocyclischen $C_{3-8}$-Ring darstellt;

X und Y unabhängig ausgewählt sind aus:

a) OH;
b) Halogen;
c) Trifluormethyl;
d) $C_{1-3}$-Alkoxy;
e) $C_{1-3}$-Alkylcarbonyloxy;
f) Phenylcarbonyloxy;
g) $C_{1-3}$-Alkoxycarbonyl;
h) Phenyloxycarbonyl;
i) Wasserstoff;
j) $C_{1-5}$-Alkyl;

T für H, tert-Butyldimethylsilyl, tert-Butyldiphenylsilyl, Trimethylsilyl, Triethylsilyl, Triisopropylsilyl oder Tetrahydropyranyl steht;

Halogen Cl oder F bedeutet,
a für eine Einfach- oder Doppelbindung steht; das die Behandlung einer Verbindung

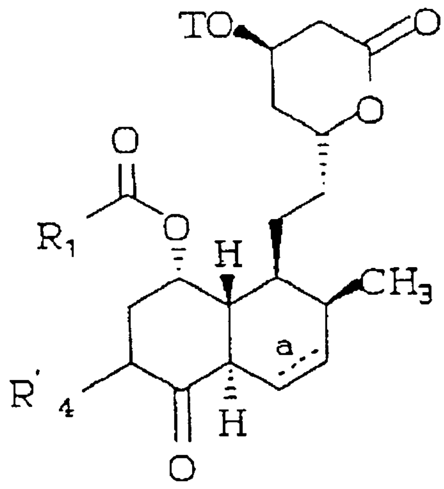

(1-5)

mit Natriumborhydrid unter Bildung von Verbindung (1-6) umfaßt und gegebenenfalls wo T nicht für H steht, die Entfernung der Hydroxy-Schutzgruppe durch Behandlung mit Tetrabutylammoniumfluorid und Essigsäure unter Bildung einer Verbindung (1-6)

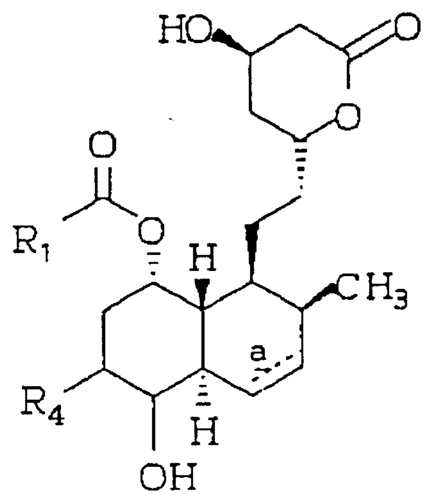

(1-6)

umfaßt, worin

$R_4$ für $R'_4$ steht, wo T für H steht.

**11.** Verwendung einer Verbindung nach Anspruch 1 zur Herstellung eines Medikaments zur Behandlung von Arteriosklerose, familiärer Hypercholesterinämie oder Hyperlipidämie.

## Revendications

**1.** Un composé représenté par les formules développées (I) ou (II):

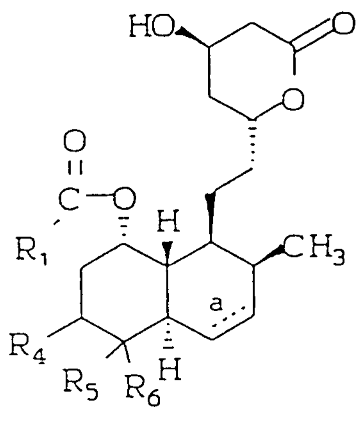

(I)

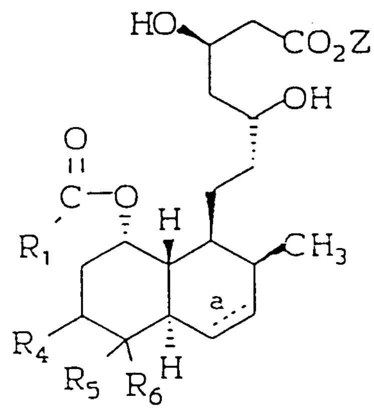

(II)

dans lesquelles:

$R_1$ est choisi parmi:

(1) un groupe alkyle en $C_1$-$C_{10}$;

(2) un groupe alkyle en $C_1$-$C_{10}$ substitué, dans lequel un ou plusieurs substituant(s) sont choisis parmi:

- (a) un atome d'halogène,
- (b) un groupe hydroxy,
- (c) un groupe alcoxy en $C_1$-$C_{10}$;
- (d) un groupe alcoxycarbonyle en $C_1$-$C_5$,
- (e) un groupe acyloxy en $C_1$-$C_5$,
- (f) un groupe cycloalkyle en $C_3$-$C_8$,
- (g) un groupe phényle,
- (h) un groupe phényle substitué dans lequel les substituants sont X et Y,
- (i) un groupe alkyl-$S(O)_n$ en $C_1$-$C_{10}$, dans lequel n a une valeur de 0 à 2,
- (j) un groupe cycloalkyl-$S(O)_n$ en $C_3$-$C_8$,
- (k) un groupe phényl-$S(O)_n$,
- (l) un groupe phényl-$S(O)_n$ substitué dans lequel les substituants sont X et Y, et
- (m) un groupe oxo;

(3) un groupe alcoxy en $C_1$-$C_{10}$;

(4) un groupe alcényle en $C_2$-$C_{10}$;

(5) un groupe cycloalkyle en $C_3$-$C_8$;

(6) un groupe cycloalkyle en $C_3$-$C_8$ substitué dans lequel un substituant est choisi parmi

- (a) un groupe alkyle en $C_1$-$C_{10}$;
- (b) un groupe alkyle en $C_1$-$C_{10}$ substitué, dans lequel un ou plusieurs substituant(s) sont choisis parmi:
  - (i) un atome d'halogène,
  - (ii) un groupe hydroxy,
  - (iii) un groupe alcoxy en $C_1$-$C_{10}$;
  - (iv) un groupe alcoxycarbonyle en $C_1$-$C_5$,
  - (v) un groupe acyloxy en $C_1$-$C_5$,
  - (vi) un groupe phényle,

(vii) un groupe phényle substitué dans lequel les substituants sont X et Y,
(viii) un groupe alkyl-$S(O)_n$ en $C_1$-$C_{10}$,
(ix) un groupe cycloalkyl-$S(O)_n$ en $C_3$-$C_8$,
(x) un groupe phényl-$S(O)_n$,
(xi) un groupe phényl-$S(O)_n$ substitué dans lequel les substituants sont X et Y, etr
(xii) un groupe oxo;
(c) un groupe alkyl-$S(O)_n$ en $C_1$-$C_{10}$,
(d) un groupe cycloalkyl-$S(O)_n$ en $C_3$-$C_8$,
(e) un groupe phényl-$S(O)_n$, (f) un groupe phényl-$S(O)_n$ substitué dans lequel les
substituants sont X et Y, et
(g) un atome d'halogène,
(h) un groupe hydroxy,
(i) un groupe alcoxy en $C_1$-$C_{10}$,
(j) un groupe alcoxycarbonyle en $C_1$-$C_5$,
(k) un groupe acyloxy en $C_1$-$C_5$,
(l) un groupe phényle, et
(m) un groupe phényle substitué dans lequel les substituants sont X et Y;
(7) un groupe phényle,
(8) un groupe phényle substitué dans lequel les substituants sont X et Y;
(9) un groupe amino;
(10) un groupe alkylamino en $C_1$-$C_5$;
(11) un groupe di(alkyl en $C_1$-$C_5$)amino;
(12) un groupe phénylamino;
(13) un groupe phénylamino substitué dans lequel les substituants sont X et Y;
(14) un groupe phényl(alkyl en $C_1$-$C_{10}$)amino;
(15) un groupe phényl(alkyl en $C_1$-$C_{10}$)amino substitué dans lequel les substituants sont X et Y.
(16) un radical choisi parmi
(a) un groupe pipéridinyle,
(b) un groupe pyrrolidinyle,
(c) un groupe pipérazinyle,
(d) un groupe morpholinyle; et
(e) un groupe thiomorpholinyle; et
(17) $R_{10}S$ dans lequel $R_{10}$ est choisi parmi
(a) un groupe alkyle en $C_1$-$C_{10}$,
(b) un groupe phényle, et
(c) un groupe phényle substitué dans lequel les substituants sont X et Y;

$R_4$ est:

(1) un atome d'hydrogène;
(2) un groupe alkyle en $C_1$-$C_{10}$; et
(3) un groupe alkyle en $C_1$-$C_{10}$ substitué, dans lequel un ou plusieurs substituant(s) sont choisis parmi:
(a) un atome d'halogène,
(b) un groupe hydroxy,
(c) un groupe alcoxy en $C_1$-$C_{10}$;
(d) un groupe alcoxycarbonyle en $C_1$-$C_5$,
(e) un groupe alkylacyloxy en $C_1$-$C_5$,
(f) un groupe phénylacyloxy,
(g) un groupe phénoxycarbonyle,
(h) un groupe phényl(alkyl en $C_1$-$C_5$)acyloxy,
(i) un groupe phényl(alcoxy en $C_1$-$C_5$),
(j) un groupe amino,

(k) un groupe alkylamino en $C_1$-$C_5$,

(l) un groupe di(alkyl en $C_1$-$C_5$)amino,

(m) un groupe phénylamino,

(n) un groupe phénylamino substitué dans lequel les substituants sont X et Y;

(o) un groupe phényl(alkyl en $C_1$-$C_5$)amino,

(p) un groupe phényl(alkyl en $C_1$-$C_5$)amino substitué dans lequel les substituants sont X et Y;

(q) un groupe cycloalkyle en $C_3$-$C_8$,

(r) un groupe phényle,

(s) un groupe phényle substitué dans lequel les substituants sont X et Y,

(t) un groupe phényl-$S(O)_n$,

(u) un groupe phényl-$S(O)_n$ substitué dans lequel les substituants sont X et Y,

(v) un groupe phényl(alkyl en $C_1$-$C_5$)-$S(O)_n$,

(w) un groupe alkyl(en $C_1$-$C_5$)-$S(O)_n$;

(x) un groupe phénylaminoacyloxy,

(y) un groupe alkyl(en $C_1$-$C_5$)aminoacyloxy,

(z) un groupe alkyl(en $C_1$-$C_5$)acylamino,

(aa) un groupe di(phényl-alkyl en $C_1$-$C_5$)phosphonyle,

(bb) un groupe di(alkyl en $C_1$-$C_5$)phosphinyle,

(4) $R_4$, avec l'atome de carbone auquel il est fixé, représente un noyau carbocyclique en $C_3$-$C_8$;

$R_5$ et $R_6$ représentent indépendamment H, OH, $OR_7$ ou $R_5$ et $R_6$, avec l'atome de carbone auquel ils sont fixés, représentent C = O, ou $R_5$ et $R_6$, avec l'atome de carbone auquel ils sont fixés, représentent un noyau carbocyclique de 3 à 7 atomes de carbone; à condition que, lorsque $R_5$ est H, $R_6$ soit OH ou $OR_7$ et que, lorsque $R_5$ est OH, $R_6$ soit H et que, lorsque $R_5$ est $OR_7$, $R_6$ soit H;

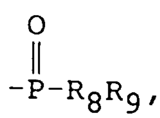, 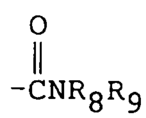 ou 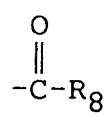, 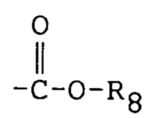,

$R_7$ est un groupe phényl(alkyle en $C_1$-$C_3$), alkyle en $C_1$-$C_5$;

$R_8$ et $R_9$ représentent indépendamment H, un groupe alkyle en $C_1$-$C_3$, phényl(alkyle en $C_1$-$C_3$) ou aryle, dans lequel le groupe aryle est un groupe phényle, naphtyle, pyridyle, furanyle, thiényle ou phényle, naphtyle, pyridyle, furanyle ou thiényle substitué par des groupes X et Y; à condition que, lorsque $R_7$ est

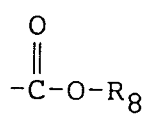,

$R_8$ ne soit pas H et que, lorsque $R_7$ est

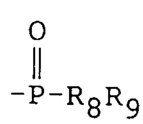,

ni $R_8$ ni $R_9$ ne soient H;

X et Y sont chacun choisis indépendamment parmi:

a) OH,

b) un atome d'halogène,
c) un groupe trifluorométhyle,
d) un groupe alcoxy en $C_1$-$C_3$,
e) un groupe alkyl(en $C_1$-$C_3$)carbonyloxy,
f) un groupe phénylcarbonyloxy,
g) un groupe alcoxy(en $C_1$-$C_3$)carbonyle,
h) un groupe phényloxycarbonyle,
i) un atome d'hydrogène,
j) un groupe alkyle en $C_1$-$C_5$;

Z est choisi parmi:
(1) un atome d'hydrogène;
(2) un groupe alkyle en $C_1$-$C_5$;
(3) un groupe en $C_1$-$C_5$ substitué dans lequel le substituant est choisi parmi:
(a) un groupe phényle,
(b) un groupe diméthylamino, et
(c) un groupe acétylamino, et
(4) un groupe 2,3-dihydroxypropyle;

l'atome d'halogène est Cl ou F;

a est une simple liaison ou une double liaison; à condition que, lorsque a est une double liaison et $R_5$ ou $R_6$ est OH, la configuration de la position 5 du noyau naphtyle soit 5(R); ou un de ses sels pharmaceutiquement acceptables.

**2.** Composé selon la revendication 1, dans lequel:

$R_4$ représente:
(1) un atome d'hydrogène;
(2) un groupe alkyle en $C_1$-$C_{10}$;
(3) un groupe alkyle en $C_1$-$C_{10}$ substitué par un ou plusieurs substituants choisis parmi:
(a) un atome d'halogène,
(b) un groupe hydroxy,
(c) un groupe amino;
(4) $CH_2R_{12}$ où $R_{12}$ est choisi parmi:
(a) un groupe alcoxy en $C_1$-$C_5$,
(b) un groupe alcoxy(en $C_1$-$C_5$)carbonyle,
(c) un groupe alkyl(en $C_1$-$C_5$)acyloxy,
(d) un groupe phénylacyloxy,
(e) un groupe phénoxycarbonyle,
(f) un groupe phényl(en $C_1$-$C_5$),
(g) un groupe phényl(alcoxy en $C_1$-$C_5$),
(h) un groupe alkylamino en $C_1$-$C_5$,
(i) un groupe di(alkyl en $C_1$-$C_5$)amino,
(j) un groupe phénylamino,
(k) un groupe phénylamino substitué dans lequel les substituants sont X et Y,
(l) un groupe phényl(alkyl en $C_1$-$C_5$)amino,
(m) un groupe phényl(alkyl en $C_1$-$C_5$)amino substitué dans lequel les substituants sont X et Y,
(n) un groupe cycloalkyle en $C_3$-$C_8$,
(o) un groupe phényle,
(p) un groupe phényle substitué dans lequel les substituants sont X et Y,
(q) un groupe phényl-$S(O)_n$,
(r) un groupe phényl-$S(O)_n$ substitué dans lequel les substituants sont X et Y,
(s) un groupe phényl(alkyl en $C_1$-$C_5$)-$S(O)_n$,
(t) un groupe alkyl(en $C_1$-$C_5$)-$S(O)_n$,
(u) un groupe phénylaminoacyloxy;
(v) un groupe alkyl(en $C_1$-$C_5$)aminoacyloxy,

(w) un groupe alkyl(en $C_1$-$C_5$)acylamino,
(x) un groupe di(phényl-alkyl en $C_1$-$C_5$)phosphonyle,
(y) un groupe di(alkyl en $C_1$-$C_5$)phosphinyle;

(5) $R_4$, avec l'atome de carbone auquel il et fixé, représente un noyau cyclopropane;

$R_5$ et $R_6$ représentent indépendamment H, OH, $OR_7$, ou $R_5$ et $R_6$, avec l'atome de carbone auquel ils sont fixés, représentent C=O, ou $R_5$ et $R_6$, avec l'atome de carbone auquel ils sont fixés, représentent un noyau cyclopropane; à condition que, lorsque $R_5$ est H, $R_6$ soit OH ou $OR_7$ et que, lorsque $R_5$ est OH, $R_6$ soit H et que, lorsque $R_5$ est $OR_7$, $R_6$ soit H;

X et Y sont choisis indépendamment parmi:

a) OH,
b) F,
c) un groupe trifluorométhyle,
d) un groupe alcoxy en $C_1$-$C_3$,
e) un atome d'hydrogène,
f) un groupe alkyle en $C_1$-$C_5$.

**3.** Composé selon la revendication 2, dans lequel:

$R_1$ est un groupe alkyle en $C_1$-$C_{10}$; et
$R_4$ est $CH_3$, H ou $CH_2$-phényle;
$R_7$ est

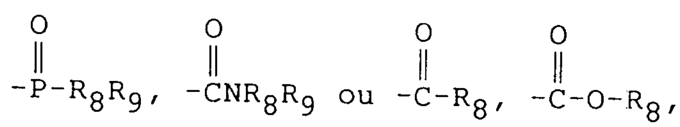

un groupe ou alkyle en $C_1$-$C_5$, phényl(alkyle en $C_1$-$C_3$);

$R_8$ et $R_9$ sont chacun indépendamment H, un groupe alkyle en $C_1$-$C_3$, un groupe phényl(alkyle en $C_1$-$C_3$) ou aryle, dans lequel le groupe aryle est un groupe phényle ou naphtyle, ou phényle ou naphtyle substitué par X.

**4.** Composé selon la revendication 3, dans lequel:

$R_1$ est un groupe 2-butyle ou 2-méthyl-2-butyle;
$R_4$ est $CH_3$.

**5.** Composé selon la revendication 2, dans lequel:

$R_1$ est un groupe alkyle en $C_1$-$C_{10}$;
$R_4$ est $CH_2OH$ ou un groupe phénylacyloxyméthyle;
$R_7$ est

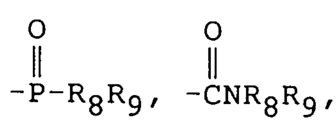

un groupe alkyle en $C_1$-$C_5$ ou phényl(alkyle en $C_1$-$C_5$);

$R_8$ et $R_9$ sont indépendamment H, un groupe alkyle en $C_1$-$C_3$, phényl(alkyle en $C_1$-$C_3$) ou aryle, dans lequel le groupe aryle est un groupe phényle ou naphtyle, ou phényle ou naphtyle substitué par X.

**6.** Composé selon la revendication 5, dans lequel:

$R_1$ est un groupe 2-butyle ou 2-méthyl-2-butyle;
$R_4$ est $CH_2OH$.

**7.** Composé selon la revendication 4, choisi parmi:
la 6(R)-[2-[8(S)-(2,2-diméthylbutyryloxy)-2(S)-méthyl-5(R)-hydroxy-6(R)-méthyl-1,2,3,4,4a(R)-,5,6,7,8,8a(R)-décahydronaphtyl-1(S)]éthyl]-4(R)-hydroxy-3,4,5,6-tétrahydro-2H-pyran-2-one;
la 6(R)-[2-[8(S)-(2,2-diméthylbutyryloxy)-2(S)-méthyl-5(S)-hydroxy-6(S)-méthyl-1,2,3,4,4a(R)-,5,6,7,8,8a(R)-décahydronaphtyl-1(S)]éthyl]-4(R)-hydroxy-3,4,5,6-tétrahydro-2H-pyran-2-one;
la 6(R)-[2-[8(S)-(2,2-diméthylbutyryloxy)-2(S)-méthyl-5(R)-benzylaminocarbonyloxy-6(R)-méthyl-1,2,3,4,4a(R),5,6,7,8,8a(R)-décahydronaphtyl-1(S)]éthyl]-4(R)-hydroxy-3,4,5,6-tétrahydro-2H-pyran-2-one;
la 6(R)-[2-[8(S)-(2,2-diméthylbutyryloxy)-2(S)-méthyl-5(R)-diphénylphosphinyloxy-6(R)-méthyl-1,2,3,4,4a(R),5,6,7,8,8a(R)-décahydronaphtyl-1(S)]éthyl]-4(R)-hydroxy-3,4,5,6-tétrahydro-2H-pyran-2-one;
la 6(R)-[2-[8(S)-(2,2-diméthylbutyryloxy)-2(S)-méthyl-5(R)-(1-phényléthyl-2-oxy)-6(R)-méthyl-1,2,3,4,4a(R),5,6,7,8,8a(R)-décahydronaphtyl-1(S)]éthyl]-4(R)-hydroxy-3,4,5,6-tétrahydro-2H-pyran-2-one;
la 6(R)-[2-[8(S)-(2,2-diméthylbutyryloxy)-2(S)-méthyl-5(R)-dibenzylaminocarbonyloxy-6(R)-méthyl-1,2,3,4,4a(R),5,6,7,8,8a(R)-décahydronaphtyl-1(S)]éthyl]-4(R)-hydroxy-3,4,5,6-tétrahydro-2H-pyran-2-one;
la 6(R)-[2-[8(S)-(2,2-diméthylbutyryloxy)-2(S)-méthyl-5(R)-hydroxy-6(S)-méthyl-1,2,3,4,4a(R)-,5,6,7,8,8a(R)-décahydronaphtyl-1(S)]éthyl]-4(R)-hydroxy-3,4,5,6-tétrahydro-2H-pyran-2-one;
la 6(R)-[2-[8(S)-(2,2-diméthylbutyryloxy)-2(S)-méthyl-5(R)-hydroxy-6(R)-méthyl-1,2,4a(R),5,6,7,8,8a-(R)-octahydronaphtyl-1(S)éthyl]-4(R)-hydroxy-3,4,5,6-tétrahydro-2H-pyran-2-one;
la 6(R)-[8(S)-(2,2-diméthylbutyryloxy)-2(S)-méthyl-5-oxo-6(R)-méthyl-1,2,3,4,4a(R),6,7,8,8a(R)-nonahydronaphtyl-1(S)]éthyl]-4(R)-hydroxy-3,4,5,6-tétrahydro-2H-pyran-2-one;
la 6(R)-[2-[8(S)-(2,2-diméthylbutyryloxy)-2(S)-méthyl-5-oxo-6(S)-méthyl-1,2,3,4,4a(R),6,7,8,8a(R)-nonahydronaphtyl-1(S)]éthyl]-4(R)-hydroxy-3,4,5,6-tétrahydro-2H-pyran-2-one; et le dihydroxyacide à noyau ouvert correspondant, et ses esters.

**8.** Composition pharmaceutique hypocholestérolémique, hypolipidémique, comprenant un véhicule pharmaceutiquement acceptable et une quantité efficace en thérapeutique d'un composé selon la revendication 1.

**9.** Composition selon la revendication 8, dans laquelle le composé est choisi parmi:
la 6(R)-[2-[8(S)-(2,2-diméthylbutyryloxy)-2(S)-méthyl-5(R)-hydroxy-6(R)-méthyl-1,2,3,4,4a(R)-,5,6,7,8,8a(R)-décahydronaphtyl-1(S)]éthyl]-4(R)-hydroxy-3,4,5,6-tétrahydro-2H-pyran-2-one;
la 6(R)-[2-[8(S)-(2,2-diméthylbutyryloxy)-2(S)-méthyl-5(S)-hydroxy-6(S)-méthyl-1,2,3,4,4a(R)-,5,6,7,8,8a(R)-décahydronaphtyl-1(S)]éthyl]-4(R)-hydroxy-3,4,5,6-tétrahydro-2H-pyran-2-one;
la 6(R)-[2-[8(S)-(2,2-diméthylbutyryloxy)-2(S)-méthyl-5(R)-benzylaminocarbonyloxy-6(R)-méthyl-1,2,3,4,4a(R),5,6,7,8,8a(R)-décahydronaphtyl-1(S)]éthyl]-4(R)-hydroxy-3,4,5,6-tétrahydro-2H-pyran-2-one;
la 6(R)-[2-[8(S)-(2,2-diméthylbutyryloxy)-2(S)-méthyl-5(R)-diphénylphosphinyloxy-6(R)-méthyl-1,2,3,4,4a(R),5,6,7,8,8a(R)-décahydronaphtyl-1(S)]éthyl]-4(R)-hydroxy-3,4,5,6-tétrahydro-2H-pyran-2-one;
la 6(R)-[2-[8(S)-(2,2-diméthylbutyryloxy)-2(S)-méthyl-5(R)-(1-phényléthyl-2-oxy)-6(R)-méthyl-1,2,3,4,4a(R),5,6,7,8,8a(R)-décahydronaphtyl-1(S)]éthyl]-4(R)-hydroxy-3,4,5,6-tétrahydro-2H-pyran-2-one;
la 6(R)-[2-[8(S)-(2,2-diméthylbutyryloxy)-2(S)-méthyl-5(R)-dibenzylaminocarbonyloxy-6(R)-méthyl-1,2,3,4,4a(R),5,6,7,8,8a(R)-décahydronaphtyl-1(S)]éthyl]-4(R)-hydroxy-3,4,5,6-tétrahydro-2H-pyran-2-one;
la 6(R)-[8(S)-(2,2-diméthylbutyryloxy)-2(S)-méthyl-5-oxo-6(R)-méthyl-1,2,3,4,4a(R),6,7,8,8a(R)-nonahydronaphtyl-1(S)]éthyl]-4(R)-hydroxy-3,4,5,6-tétrahydro-2H-pyran-2-one;
la 6(R)-[2-[8(S)-(2,2-diméthylbutyryloxy)-2(S)-méthyl-5-oxo-6(S)-méthyl-1,2,3,4,4a(R),6,7,8,8a(R)-nonahydronaphtyl-1(S)]éthyl]-4(R)-hydroxy-3,4,5,6-tétrahydro-2H-pyran-2-one;
la 6(R)-[2-[8(S)-(2,2-diméthylbutyryloxy)-2(S)-méthyl-5(R)-hydroxy-6(S)-méthyl-1,2,3,4,4a(R)-,5,6,7,8,8a(R)-décahydronaphtyl-1(S)]éthyl]-4(R)-hydroxy-3,4,5,6-tétrahydro-2H-pyran-2-one;
la 6(R)-[2-[8(S)-(2,2-diméthylbutyryloxy)-2(S)-méthyl-5(R)-hydroxy-6(R)-méthyl-1,2,4a(R),5,6,7,8,8a-(R)-décaoctahydronaphtyl-1(S)]éthyl]-4(R)-hydroxy-3,4,5,6-tétrahydro-2H-pyran-2-one; et le dihydroxyacide à noyau ouvert correspondant, et ses esters.

**10.** Procédé de formation d'un composé (1-6):

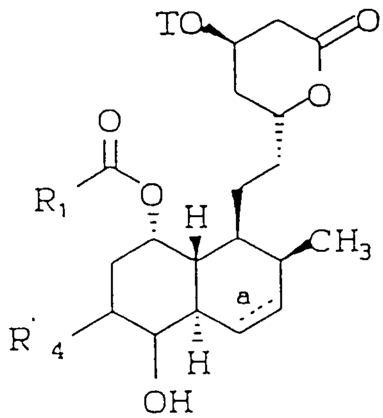

(1-6)

dans laquelle

$R_1$ est choisi parmi:

(1) un groupe alkyle en $C_1$-$C_{10}$;

(2) un groupe alkyle en $C_1$-$C_{10}$ substitué, dans lequel un ou plusieurs substituant(s) sont choisis parmi:

(a) un atome d'halogène,

(b) un groupe hydroxy,

(c) un groupe alcoxy en $C_1$-$C_{10}$;

(d) un groupe alcoxycarbonyle en $C_1$-$C_5$,

(e) un groupe acyloxy en $C_1$-$C_5$,

(f) un groupe cycloalkyle en $C_3$-$C_8$,

(g) un groupe phényle,

(h) un groupe phényle substitué dans lequel les substituants sont X et Y,

(i) un groupe alkyl-$S(O)_n$ en $C_1$-$C_{10}$, dans lequel n a une valeur de 0 à 2,

(j) un groupe cycloalkyl-$S(O)_n$ en $C_3$-$C_8$,

(k) un groupe phényl-$S(O)_n$,

(l) un groupe phényl-$S(O)_n$ substitué dans lequel les substituants sont X et Y, et

(m) un groupe oxo;

(3) un groupe alcoxy en $C_1$-$C_{10}$;

(4) un groupe alcényle en $C_2$-$C_{10}$;

(5) un groupe cycloalkyle en $C_3$-$C_8$;

(6) un groupe cycloalkyle en $C_3$-$C_8$ substitué dans lequel un substituant est choisi parmi

(a) un groupe alkyle en $C_1$-$C_{10}$;

(b) un groupe alkyle en $C_1$-$C_{10}$ substitué, dans lequel un ou plusieurs substituant(s) sont choisis parmi:

(i) un atome d'halogène,

(ii) un groupe hydroxy,

(iii) un groupe alcoxy en $C_1$-$C_{10}$;

(iv) un groupe alcoxycarbonyle en $C_1$-$C_5$,

(v) un groupe acyloxy en $C_1$-$C_5$,

(vi) un groupe phényle,

(vii) un groupe phényle substitué dans lequel les substituants sont X et Y,

(viii) un groupe alkyl-$S(O)_n$ en $C_1$-$C_{10}$,

(ix) un groupe cycloalkyl-$S(O)_n$ en $C_3$-$C_8$,
(x) un groupe phényl-$S(O)_n$,
(xi) un groupe phényl-$S(O)_n$ substitué dans lequel les substituants sont X et Y, et
(xii) un groupe oxo;
(c) un groupe alkyl-$S(O)_n$ en $C_1$-$C_{10}$,
(d) un groupe cycloalkyl-$S(O)_n$ en $C_3$-$C_8$,
(e) un groupe phényl-$S(O)_n$,
(f) un groupe phényl-$S(O)_n$ substitué dans lequel les substituants sont X et Y, et
(g) un atome d'halogène,
(h) un groupe hydroxy,
(i) un groupe alcoxy en $C_1$-$C_{10}$,
(j) un groupe alcoxycarbonyle en $C_1$-$C_5$,
(k) un groupe acyloxy en $C_1$-$C_5$,
(l) un groupe phényle, et
(m) un groupe phényle substitué dans lequel les substituants sont X et Y;
(7) un groupe phényle,
(8) un groupe phényle substitué dans lequel les substituants sont X et Y;
(9) un groupe amino;
(10) un groupe alkylamino en $C_1$-$C_5$;
(11) un groupe di(alkyl en $C_1$-$C_5$)amino;
(12) un groupe phénylamino;
(13) un groupe phénylamino substitué dans lequel les substituants sont X et Y;
(14) un groupe phényl(alkyl en $C_1$-$C_{10}$)amino;
(15) un groupe phényl(alkyl en $C_1$-$C_{10}$)amino substitué dans lequel les substituants sont X et Y;
(16) un radical choisi parmi
(a) un groupe pipéridinyle,
(b) un groupe pyrrolidinyle,
(c) un groupe pipérazinyle,
(d) un groupe morpholinyle,
(e) un groupe thiomorpholinyle; et
(17) $R_{10}S$ dans lequel $R_{10}$ est choisi parmi
(a) un groupe alkyle en $C_1$-$C_{10}$,
(b) un groupe phényle, et
(c) un groupe phényle substitué dans lequel les substituants sont X et Y;

$R'_4$ est:

(1) un atome d'hydrogène;
(2) un groupe alkyle en $C_1$-$C_{10}$; et
(3) un groupe alkyle en $C_1$-$C_{10}$ substitué, dans lequel un ou plusieurs substituant(s) sont choisis parmi:
(a) un atome d'halogène,
(b) OT,
(c) un groupe alcoxy en $C_1$-$C_{10}$;
(d) un groupe alcoxycarbonyle en $C_1$-$C_5$,
(e) un groupe alkylacyloxy en $C_1$-$C_5$,
(f) un groupe phénylacyloxy,
(g) un groupe phénoxycarbonyle,
(h) un groupe phényl(alkyl en $C_1$-$C_5$)acyloxy,
(i) un groupe phényl(alcoxy en $C_1$-$C_5$),
(j) un groupe amino,
(k) un groupe alkylamino en $C_1$-$C_5$,
(l) un groupe di(alkyl en $C_1$-$C_5$)amino,
(m) un groupe phénylamino,

(n) un groupe phénylamino substitué dans lequel les substituants sont X et Y;
(o) un groupe phényl(alkyl en $C_1$-$C_5$)amino,
(p) un groupe phényl(alkyl en $C_1$-$C_5$)amino substitué dans lequel les substituants sont X et Y;
(q) un groupe cycloalkyle en $C_3$-$C_8$,
(r) un groupe phényle,
(s) un groupe phényle substitué dans lequel les substituants sont X et Y,
(t) un groupe phényl-$S(O)_n$,
(u) un groupe phényl-$S(O)_n$ substitué dans lequel les substituants sont X et Y,
(v) un groupe phényl(alkyl en $C_1$-$C_5$)-$S(O)_n$,
(w) un groupe alkyl(en $C_1$-$C_5$)-$S(O)_n$;
(x) un groupe phénylaminoacyloxy,
(y) un groupe alkyl(en $C_1$-$C_5$)aminoacyloxy,
(z) un groupe alkyl(en $C_1$-$C_5$)acylamino,
(aa) un groupe di(phényl-alkyl en $C_1$-$C_5$)phosphonyle,
(bb) un groupe di(alkyl en $C_1$-$C_5$)phosphinyle,

(4) R'$_4$, avec l'atome de carbone auquel il est fixé, représente un noyau carbocyclique en $C_3$-$C_8$;

X et Y sont chacun choisis indépendamment parmi:
a) OH,
b) un atome d'halogène,
c) un groupe trifluorométhyle,
d) un groupe alcoxy en $C_1$-$C_3$,
e) un groupe alkyl(en $C_1$-$C_3$)carbonyloxy,
f) un groupe phénylcarbonyloxy,
g) un groupe alcoxy(en $C_1$-$C_3$)carbonyle,
h) un groupe phényloxycarbonyle,
i) un atome d'hydrogène,
j) un groupe alkyle en $C_1$-$C_5$;

T est H, un groupe t-butyldiméthylsilyle, t-butyldiphénylsilyle, triméthylsilyle, triéthylsilyle, triisopropylsilyle ou tétrahydropyranyle;

l'atome d'halogène est Cl ou F;

a est une simple liaison ou une double liaison; qui comprend le traitement d'un composé

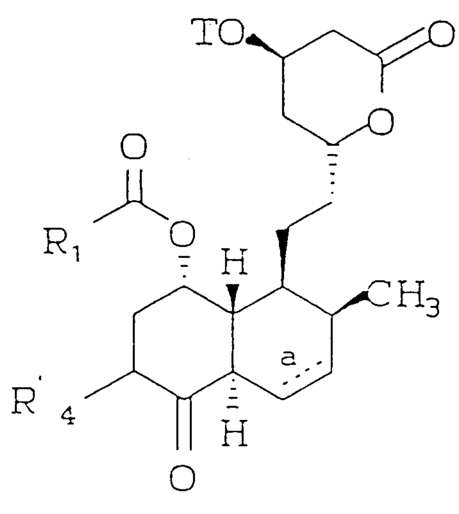

(1-5)

avec du borohydrure de sodium, pour former le composé (1-6); et éventuellement, lorsque T n'est pas H, l'élimination du groupe protecteur de fonction hydroxyle par traitement avec du fluorure de tétrabutylammonium et de l'acide acétique, pour former un composé (1-6)

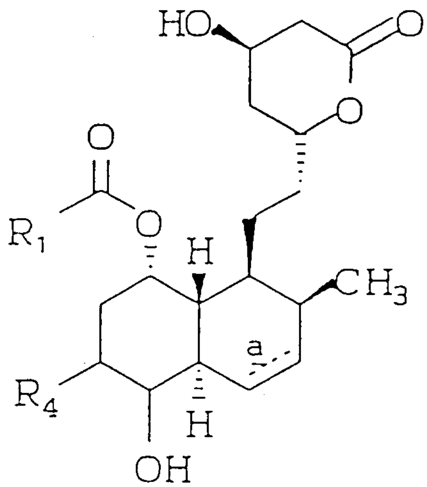

(1-6)

dans laquelle:

$R_4$ est $R'_4$ lorsque T est H.

**11.** Utilisation d'un composé selon la revendication 1 pour fabriquer un médicament servant à traiter l'artériosclérose, l'hypercholestérolémie ou l'hyperlipidémie familiale.